# EUROPEAN PATENT APPLICATION

(11) **EP 4 455 308 A1**
(43) Date of publication of application: **30.10.2024**
(21) Application number: 23169545.3
(22) Date of filing: 24.04.2023
(51) Int. Cl.: C12Q 1/6883, C12Q 1/6886

(54) **IL-7R GENE SIGNATURES**

(71) Applicant: Les Laboratoires Servier, 92284 Suresnes (FR); Institut National de la Santé et de la Recherche Médicale, 75654 Paris Cedex 13 (FR); Universite Brest Bretagne Occidentale, 29200 Brest (FR)
(72) Inventor: The designation of the inventor has not yet been filed

(57) **Abstract**

The present invention relates to IL-7R signatures useful in a method of identification of a patient suffering from IL-7R-mediated disease, a method of identification of a patient likely responsive to a treatment with an IL-7R modulator, a method for evaluating the therapeutic response to an IL-7R modulator of a patient treated with said IL-7R modulator, a method for predicting an IL-7R-mediated disease activity in a patient suffering from said disease, a method for monitoring a treatment with an IL-7R modulator, a method for selecting a compound likely effective in the treatment of a disease associated with an increase or decrease of the IL-7R signaling pathway or a kit comprising a set of reagents that specifically detects the gene expression profile of IL-7R gene signatures.

## Description

### BACKGROUND OF THE INVENTION

lnterleukin-7 (IL-7) is a limiting and non-redundant cytokine known to play an essential role in T and B cell lymphopoiesis, thymocyte maturation, and mature T cell homeostasis. IL-7 is mainly produced by epithelial and stromal cells but also by keratinocytes and dendritic cells. It is known to regulate T cell homeostatic proliferation and survival for both memory and naive T cell populations. Since conventional mature T lymphocytes express high levels of interleukin-7 receptor (IL-7R), except for naturally occurring regulatory T-cells (Tregs) that express low IL-7R, it constitutes a unique opportunity to selectively target pathogenic effectors while preserving natural regulators.

IL-7 signals through the cell-surface IL-7R, formed by the dimerization of the IL-7Rα (also known as CD127) and the common cytokine receptor gamma chain (γ- chain/CD132). IL-7Rα and the γ-chain interact together, stabilizing and forming an active IL-7/IL-7Rα/γ-chain ternary complex which is then internalized. IL-7 induces mainly the Janus activated kinase (JAK) signal transducer and activator of transcription protein 5 (STAT5) pathway, thereby delivering proliferative and anti-apoptotic signals as well as regulating expression of anti- and pro-apoptotic B-cell lymphoma-2 family (Bcl-2) members.

Disturbance of IL-7R pathway has been implicated in the pathogenesis of several chronic inflammatory diseases, including rheumatoid arthritis (RA), inflammatory bowel diseases, ulcerative colitis (UC), Crohn disease, and autoimmune diseases, including cutaneous lupus erythematosus, systemic lupus erythematosus (SLE), lupus nephritis, multiple sclerosis, primary Sjögren's syndrome (pSS) by notably driving effects on inflammatory pathways through monocytes and epithelial, T and B cells. Indeed, blocking the IL-7R pathway has been shown to reverse autoimmunity and chronic inflammation in various *in vitro* and *in vivo* models. A major challenge in the treatment of auto-immune and inflammatory diseases is the presence of pathogenic memory T cells in the tissues, which are likely resistant to conventional immune modulator therapy and tolerance strategies. IL-7R pathway is a known survival factor for memory T cells, described to be present in inflammatory diseases and autoimmune diseases.

Sjögren's syndrome (SS) is one of the most common autoimmune disorders, with an estimated prevalence of 60.82 per 100 000 inhabitants worldwide. The spectrum of the disease extends from an organ-specific autoimmune disorder to a systemic process and is also associated with an increased risk of B-cell lymphoma. In particular, SS is characterized by a hallmark of symptoms encompassing sicca symptoms (ocular, oral and/or vagina dryness), arthralgia, associated to diffuse pain and fatigue. Primary Sjögren's syndrome (pSS) refers to the absence of another connective tissue disorder, where systemic manifestations are estimated to occur in 30 to 40% of the patients, affecting different organs or systems (joint, lung, nervous system, kidney or non-exocrine glands). pSS differs from Sicca syndrome, which is characterized by oral and ocular dryness without autoimmune component like the presence of auto-antibodies nor any systemic manifestations.

The diagnosis of pSS is based upon a combination of clinical, serological, histological, and functional parameters. The 2016 American College of Rheumatology (ACR)/European League Against Rheumatism (EULAR) classification criteria request either anti-SSA (also called anti-Ro) antibodies or a positive minor salivary gland (MSG) biopsy (highly specific but invasive and requiring standardization).

Most commonly the biopsies are performed on labial salivary glands, but they can also be performed on parotid glands by an experienced clinician. The typical histopathological changes in the minor salivary glands are well-defined focal infiltrates of mostly lymphocytes surrounding ducts or small vessels. The dominating cell populations in the focal infiltrates are T and B lymphocytes. Higher focus scores are associated with an increased B/T-cell ratio.

Regarding anti-SSA antibodies, the SSA/Ro antigen includes two distinct components, i.e. SSA/Ro52 and SSA/Ro60, with SSA/Ro60 being the most common in pSS. It has been demonstrated that these auto-antibodies are present many years before the clinical onset of pSS, and the number of auto-antibodies increases during disease progression. They are also associated with worst exocrine gland function, parotid gland enlargement and several extra-glandular manifestations.

There is currently no approved treatment for primary Sjögren's syndrome. As a result of the heterogeneous presentation of the syndrome, a major challenge remains to improve diagnosis and therapy.

The main current focus of treatments in Sjögren's syndrome is symptom management with sialagogues and topical therapy, which does not address the underlying autoimmune disease processes. Importantly, a significant proportion of patients with acute systemic manifestations are treated with aggressive treatments including pan-cytopenic immunosuppressants.

Several observations in pSS patients have highlighted the potential role of the IL-7R axis in the disease pathophysiology. Levels of IL-7 and IL-7Rα, were found to be elevated in the salivary glands (SGs) of patients with an IL-7 contribution to local T cell dependent B cell activation. Serum IL-7 level was associated with B cell and IFN-related biomarkers in pSS, such as anti-SSA antibody and CXCL13. This axis was involved in the formation of tertiary lymphoid structures (TLS) in SGs, which were reported to be a predictor of lymphoma development in pSS.

IL-7 and IL-7R are also involved in the promotion of cancer. Activating mutations of IL-7R and overexpression of IL-7R have been described in acute lymphoblastic leukemia (ALL) as T- and B-ALL. Acute lymphoblastic leukemia is an aggressive cancer arising from lymphoid progenitors. Around 75-80% of cases of adult ALL are of B-cell lineage and 20-25% belong to the T-cell lineage. Improving the understanding of ALL molecular causes and underlying biology is critical to better classify patients and identify the best targeted treatment options that can improve efficacy and minimize toxicities for each particular patient subset. For instance, the genetic background of T-ALL is widely heterogeneous and biomarkers are not presently adopted in the diagnostic stage of this disease. It has been described that high expression of IL-7Rα can promote T-cell tumorigenesis, even in the absence of IL-7Rα gain-of-function mutations. Recent studies using IL-7R silencing or IL-7R antagonist demonstrated inhibition of leukemia stem cell activity. Overall, blocking the IL-7/IL-7R pathway has been shown to reverse autoimmunity and chronic inflammation as well as to present beneficial effect in cancers.

Other studies showed additional various therapeutic applications of IL-7 administration, including treatment of patients with immunodeficiency states, like HIV patients, or treatment of patients suffering from iatrogenic diseases post chemotherapy or with toxicities post hematopoietic stem cell transplantation.

The difficulty in developing effective therapies is in part due to the heterogeneity in the clinical manifestations and the pathophysiology of diseases. Therefore, there is a high need today for targeted therapies and biomarkers to select patients suffering from IL-7R-mediated diseases, such as inflammatory diseases, autoimmune diseases, in particular pSS, UC, Crohn's disease or RA, and cancers, in particular ALL, would be extremely valuable.

To this aim, identification of molecular IL-7R gene signatures reflecting the involvement of IL-7/IL-7R pathway would allow the selection of such patients and ensure they will receive the right treatment in relation with the specific pathophysiological mechanisms underlying their disease. Such precision medicine strategies will greatly improve the management of the patients' disease. In addition, IL-7R gene signatures could be used in clinical trials design as stratification markers to ensure IL-7R-modulating therapeutics are given to individuals who are most likely to benefit or have the most significant improvements to gain. Moreover, such IL-7R gene signatures would be decisive for following treatment efficacy of IL-7R therapies, using either IL-7R agonist or IL-7R antagonist. They will help to identify relapse, to define the best treatment strategy, either to continue the therapy, to move to treatment combination or to switch to another treatment.

### SUMMARY OF THE INVENTION

Dysregulation of the IL-7/IL-7R pathway can be involved in the pathophysiology of multiple diseases characterized by heterogenous clinical manifestations. Several diseases are associated with an increase or decrease of the IL-7R signaling pathway, including but not limited to autoimmune diseases, inflammatory diseases, cancer diseases and infectious diseases. There is a need today to identify patients suffering from such disease associated with an increase or decrease of the IL-7R signaling pathway, and to monitor IL-7R pathway activity in such patients.

In order to give more-effective personalized therapies to the patients and to better and easily monitor IL-7R pathway activity, more efficiently and at a lower cost, the inventors in the present application developed new powerful IL-7R signatures comprising a very limited number of genes, that can be used as highly efficient biomarkers of IL-7R pathway, to identify patient suffering from diseases associated with an increase or decrease of the IL-7R signaling pathway, to identify patients likely responsive to IL-7R modulators (i.e. IL-7R agonist or IL-7R antagonist), to monitor accurately the efficacy of IL-7R modulators treatment as well as to correctly stratify the patients based on these new IL-7R signatures.

The present invention is directed to a method of identification of a patient likely suffering from a disease associated with an increase or decrease of the IL-7R signaling pathway comprising the steps of:
a) determining the gene expression profile in a biological sample of said patient of at least 2, 3 or 4 genes selected from the group consisting of: IKZF4, KIAA0040, PGAP1 and SOS1 genes, or
b) determining the gene expression profile in a biological sample of said patient of at least 2, 3, 4 or 5 genes selected from the group consisting of: IL-7R, PCED1 B, TNFSF8, ADAM19 and MYBL1 genes, and
c) comparing the gene expression profile determined in a) or b) to a reference gene expression profile of said genes,

preferably wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells,
wherein respectively a higher or lower gene expression profile of said genes in said patient sample as compared to the reference gene expression profile of said genes is indicative that the patient is likely suffering from a disease respectively associated with an increase or decrease of the IL-7R signaling pathway, preferably wherein said disease associated with an increase or decrease of the IL-7R signaling pathway is selected from the group consisting of an autoimmune disease, an inflammatory disease, a cancer disease and an infectious disease, in particular wherein the autoimmune disease or inflammatory disease is Sjogren's syndrome or Sjogren's disease, systemic lupus erythematosus, rheumatoid arthritis, sclerosis, psoriasis, intestinal bowel disease or ulcerative colitis, in particular wherein the cancer disease is T-cell acute lymphoblastic leukemia, B-cell acute lymphoblastic leukemia, chronic lymphocytic leukemia, T-cell prolymphocytic leukemia and Hodgkin's lymphoma.

The present invention is also directed to a method of identification of a patient likely responsive to treatment with an IL-7R modulator, i.e IL-7R agonist or IL-7R antagonist, prior to said treatment by:
a) determining in a biological sample of said patient, whether the patient's gene expression profile of at least 2, 3 or 4 genes selected from the group consisting of: IKZF4, KIAA0040, PGAP1 and SOS1 genes is higher or lower compared to a reference gene expression profile, or
b) determining in a biological sample of said patient, whether the patient's gene expression profile of at least 2, 3, 4 or 5 genes selected from the group consisting of: IL-7R, PCED1B, TNFSF8, ADAM19 and MYBL1 genes is higher or lower compared to a reference gene expression profile,

preferably wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells,
wherein respectively a higher or lower gene expression profile determined in a) or b) is indicative that the patient is likely responsive to treatment with respectively an IL-7R antagonist or with an IL-7R agonist.

The present invention is also directed to a method for evaluating the therapeutic response of a patient treated with an IL-7R modulator, i.e. respectively with an IL-7R agonist or IL-7R antagonist, comprising the steps of:
a) determining in a biological sample of said patient the gene expression profile of at least 2, 3 or 4 genes selected from the group consisting of: IKZF4, KIAA0040, PGAP1 and SOS1 genes, or
b) determining in a biological sample of said patient the gene expression profile of at least 2, 3, 4 or 5 genes selected from the group consisting of: IL-7R, PCED1B, TNFSF8, ADAM19 and MYBL1 genes, and
c) comparing said gene expression profile to a gene expression profile determined in the same patient before treatment or earlier during the treatment,

preferably wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells,
wherein respectively a higher or lower gene expression profile is indicative that the patient is likely responsive to the treatment with respectively the IL-7R agonist or the IL-7R antagonist.

The present invention is also directed to a method for evaluating the therapeutic response of a patient treated with an IL-7R modulator, respectively with an IL-7R agonist or IL-7R antagonist, comprising the steps of:
a) determining in a biological sample of said patient the gene expression profile of at least 2, 3 or 4 genes selected from the group consisting of: IKZF4, KIAA0040, PGAP1 and SOS1 genes, or
b) determining in a biological sample of said patient the gene expression profile of at least 2, 3, 4 or 5 genes selected from the group consisting of: IL-7R, PCED1B, TNFSF8, ADAM19 and MYBL1 genes, and
c) comparing said gene expression profile to a gene expression profile determined in the patient before treatment or earlier during the treatment,

preferably wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells,
wherein respectively a lower or higher gene expression profile is indicative that the patient is likely not responsive to the treatment with respectively the IL-7R agonist or the IL-7R antagonist.

The present invention is also directed to a method for evaluating the therapeutic response of a patient treated with an IL-7R modulator, i.e. with an IL-7R agonist or IL-7R antagonist, comprising the steps of:
a) determining in a biological sample of said patient the gene expression profile of at least 2, 3 or 4 genes selected from the group consisting of: IKZF4, KIAA0040, PGAP1 and SOS1 genes, or
b) determining in a biological sample of said patient the gene expression profile of at least 2, 3, 4 or 5 genes selected from the group consisting of: IL-7R, PCED1B, TNFSF8, ADAM19 and MYBL1 genes, and
c) comparing said gene expression profile to a gene expression profile determined in the patient before treatment or earlier during the treatment,
wherein an unchanged gene expression profile is indicative that the patient is likely not responsive to the treatment with said IL-7R modulator.

In another aspect, the invention is directed to a method for predicting an IL-7R-mediated disease activity in a patient suffering from said disease, comprising the steps of:
a) determining, in a biological sample of said patient, the patient's gene expression profile of at least 2, 3 or 4 genes selected from the group consisting of: IKZF4, KIAA0040, PGAP1 and SOS1 genes, or
b) determining, in a biological sample of said patient, the patient's gene expression profile of at least 2, 3, 4 or 5 genes selected from the group consisting of: IL-7R, PCED1B, TNFSF8, ADAM19 and MYBL1 genes, and
c) comparing the gene expression profile determined in a) or b) to a gene expression profile of a representative group of patients suffering from said IL-7R-mediated disease,

wherein the patient's gene expression profile is indicative of a high disease activity when said profile is significantly higher than the gene expression profile of a representative group of patients with said IL-7R-mediated disease,
wherein the patient's gene expression profile is indicative of a low disease activity when said profile is significantly lower than the gene expression profile of a representative group of patients with said IL-7R-mediated disease.

In another aspect, the present invention is directed to a method for selecting a compound likely effective in the treatment of a disease associated with respectively an increase or decrease of the IL-7R signaling pathway comprising the steps of:
a) culturing cells from a biological sample,
b) determining in said cell culture, the gene expression profile of at least 2, 3 or 4 genes selected from the group consisting of: IKZF4, KIAA0040, PGAP1 and SOS1 genes, and/or determining in said cell culture the gene expression profile of at least 2, 3, 4 or 5 genes selected from the group consisting of: IL-7R, PCED1B, TNFSF8, ADAM19 and MYBL1 genes,
c) comparing the determined gene expression profile to a reference gene expression profile, and
d) selecting a compound that induces respectively a lower or higher gene expression profile of said genes in said cell culture as compared to a reference gene expression profile, in particular wherein such compound is an IL-7R modulator, respectively an IL-7R antagonist or an IL-7R agonist, in particular wherein gene expression profile is determined by detecting the mRNA expression of said genes.

In another aspect, the present invention relates to a kit comprising a set of reagents that specifically detects the gene expression profile of at least 2, 3 or 4 genes selected from the group consisting of: IKZF4, KIAA0040, PGAP1 and SOS1 genes, and/or at least 2, 3, 4 or 5 genes selected from the group consisting of: IL-7R, PCED1B, TNFSF8, ADAM19, MYBL1 genes, wherein said reagents are primer pairs and/or probes specific of each gene.

### BRIEF DESCRIPTION OF THE DRAWINGS

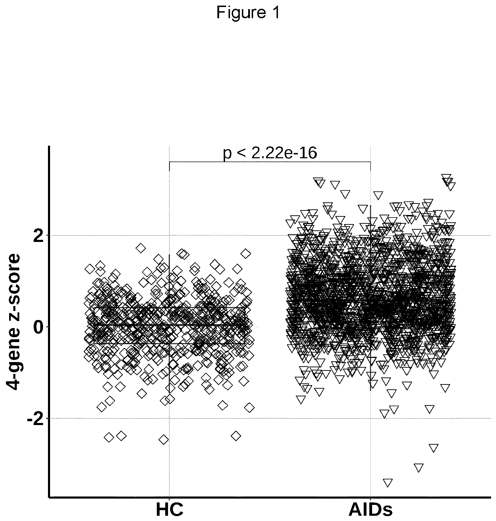

**Figure 1****:** Boxplot representing the z-score of the 4-gene signature *(IKZF4, KIAA0040, PGAP1 and* SOS1) applied on patients of different autoimmune diseases (AIDs) compared to healthy control subjects (HCs, n=555) (statistical test: Wilcoxon).

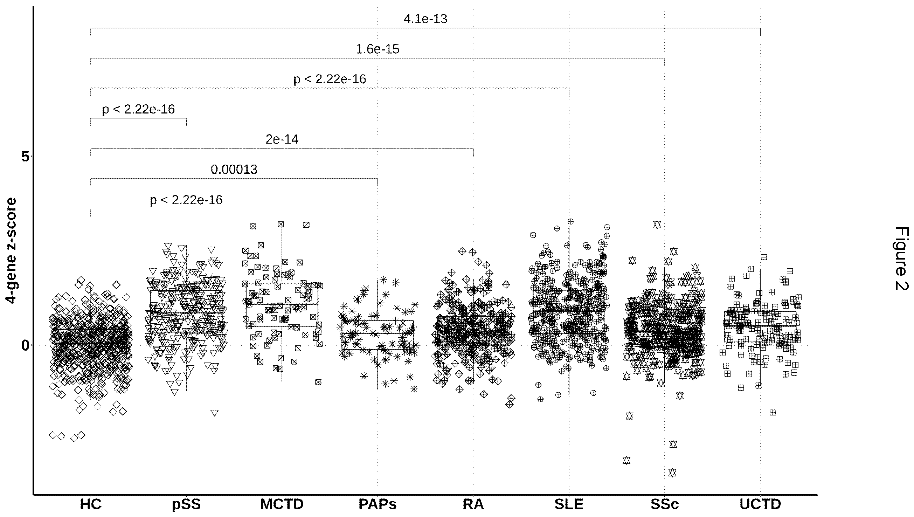

**Figure 2****:** Boxplot representing the z-score of the 4-gene signature (*IKZF4, KIAA0040, PGAP1 and SOS1*) applied on individual AIDs namely; primary Sjögren's syndrome (pSS, n=341), systemic lupus erythematosus (SLE, n=405), rheumatoid arthritis (RA, n=309), systemic sclerosis (SSc, n=328), mixed connective tissue disease (MCTD, n=91), undifferentiated connective tissue disease (UCTD, n=133) or anti-phospholipid syndrome (PAPs, n=92), when compared to healthy controls (HCs, n=555) (statistical test: Wilcoxon).

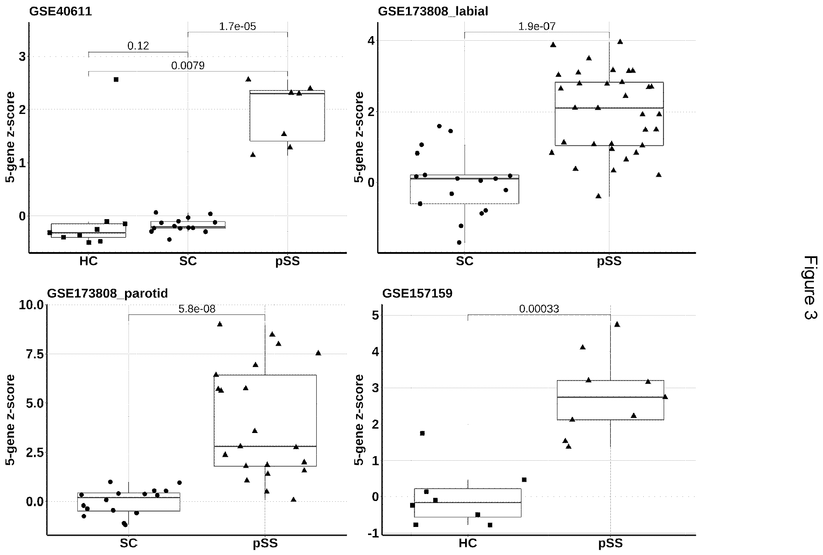

**Figure 3****:** Boxplot representing the z-score of the 5-gene signature (*IL-7R, PCED18, TNFSF8, ADAM19,* and *MYBL1)* in different salivary glands of pSS patients (triangle) versus healthy control subjects (HCs) (square) or sicca control patients (SCs) (circle) in the different tissue data sets (detailed in Table 3), statistical test: Wilcoxon.

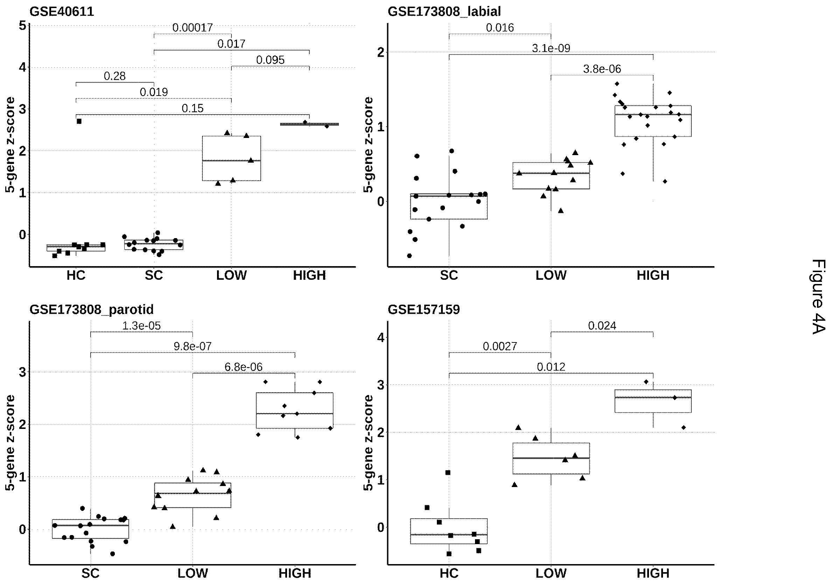

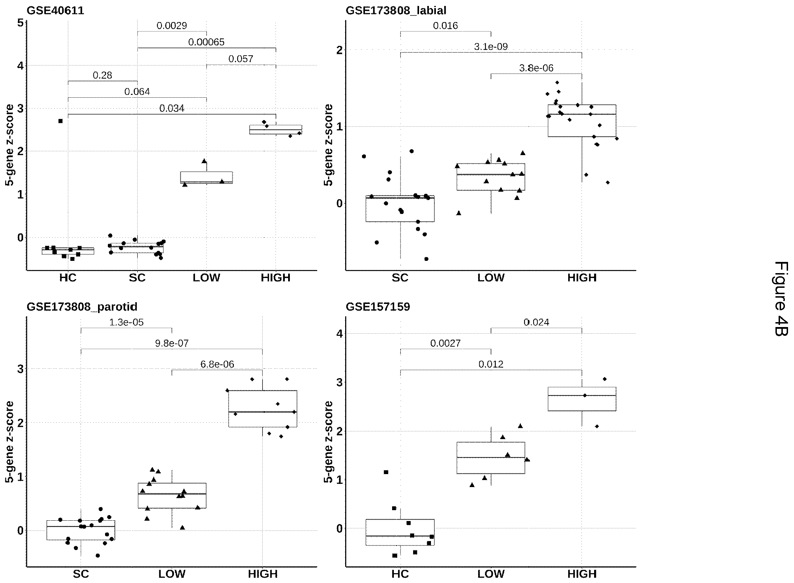

**Figure 4****:** shows the 5-gene signature IL-7R, PCED1B, TNFSF8, ADAM19, MYBL1 z-score association with the salivary glands' lymphocytes infiltration degree. **Figure 4A****:** Boxplot of the IL-7R 5 genes signature z-score *(IL-7R, PCED1B, TNFSF8, ADAM19, MYBL1)* according to B cell enrichment scores in primary Sjögren's syndrome (pSS) tissue datasets: high B cells infiltrates in pSS patients (diamond), low B cells infiltrates in pSS patients (triangle) versus sicca control patients (SCs) (circle) or healthy control subjects (HCs) (square). **Figure 4B****:** Boxplot of the 5 genes signature z-score *(IL-7R, PCED18, TNFSF8, ADAM19, MYBL1)* according to the infiltration profile compares pSS high lymphocyte infiltrates (diamond), pSS low lymphocyte infiltrates (triangle) versus sicca controls (SCs) (circle) or healthy controls (HCs) (square).

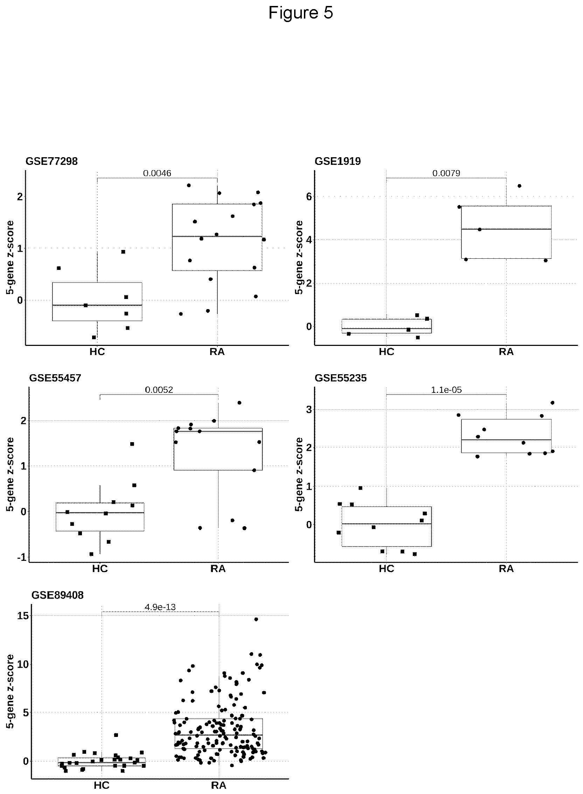

**Figure 5****:** Boxplot representing the z-score of the 5-gene signature *(IL-7R, PCED18, TNFSF8, ADAM19,* and *MYBL1)* in different data sets of synovial tissue of rheumatoid arthritis patients (triangle) versus healthy controls (HCs) (square) (data set detailed in Table 5), statistical test: Wilcoxon.

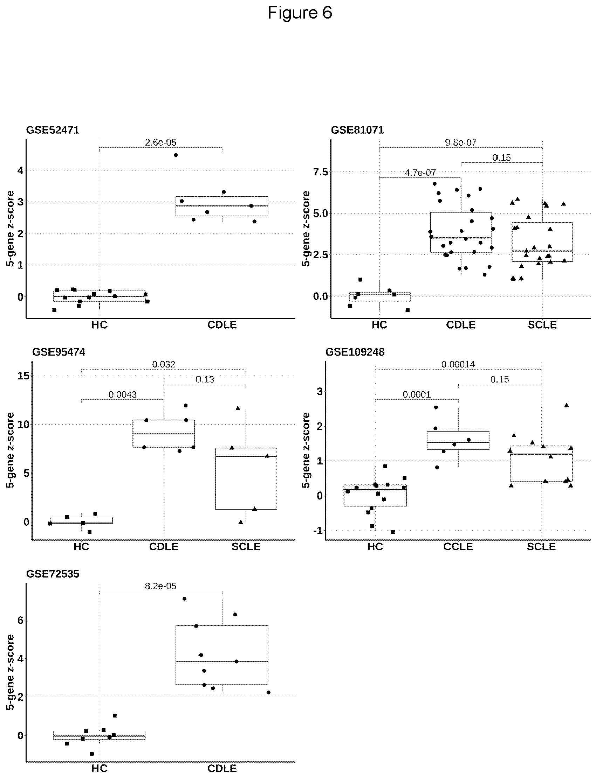

**Figure 6****:** Boxplot representing the z-score of the 5-gene signature (*IL-7R, PCED18, TNFSF8, ADAM19,* and *MYBL1)* in different data sets of skin biopsies of different types of cutaneous lupus erythematosus patients (chronic discoid lupus erythematosus (CDLE) and subacute cutaneous lupus erythematosus (sCLE)), statistical test: Wilcoxon, (data set detailed in Table 6).

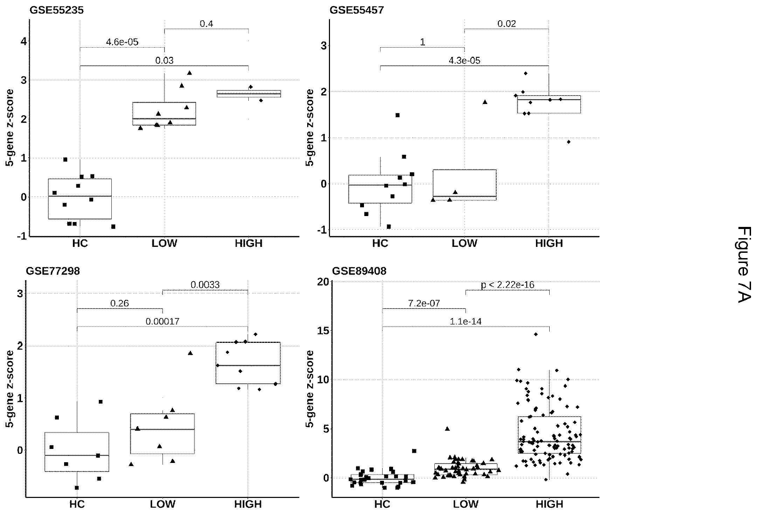

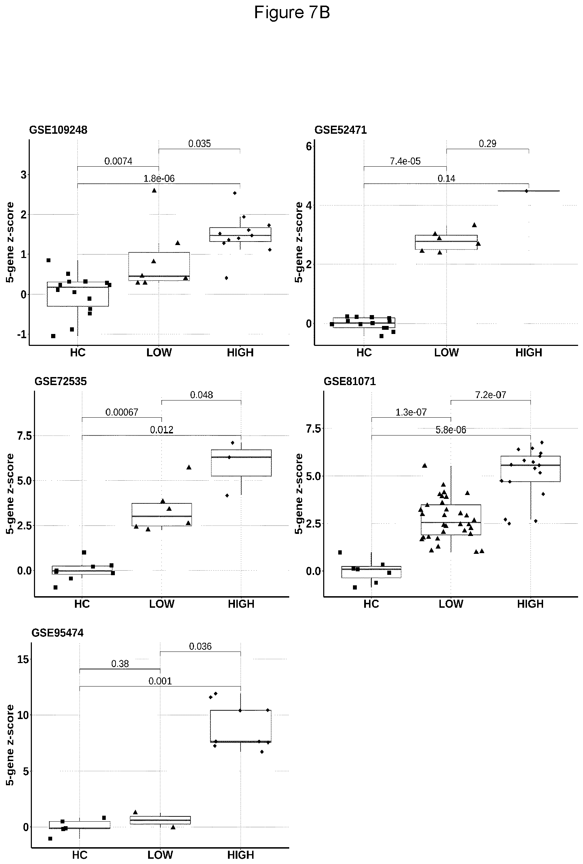

**Figure 7****:** shows the 5-gene signature (*IL-7R, PCED1B, TNFSF8, ADAM19, MYBL1*) z-score correlation with lymphocyte cells infiltration degree. **Figure 7A****:** Boxplot of the IL-7R 5 genes signature z-score (IL-7R, PCED1B, TNFSF8, ADAM19, MYBL1) according to lymphocytes enrichment scores in the synovial tissue of RA patients' datasets: RA patients high lymphocyte infiltrates (diamond), RA low lymphocyte infiltrates (triangle) versus healthy controls (HCs) (square). **Figure 7B****:** Boxplot of the IL-7R 5-gene signature z-score (IL-7R, PCED1B, TNFSF8, ADAM19, MYBL1) according to lymphocytes enrichment scores in various skin tissue of cutaneous lupus erythematosus (CLE) patients' datasets: CLE patients with high lymphocyte infiltrates (diamond), CLE patients with low lymphocyte infiltrates (triangle) versus healthy controls (HCs) (square).

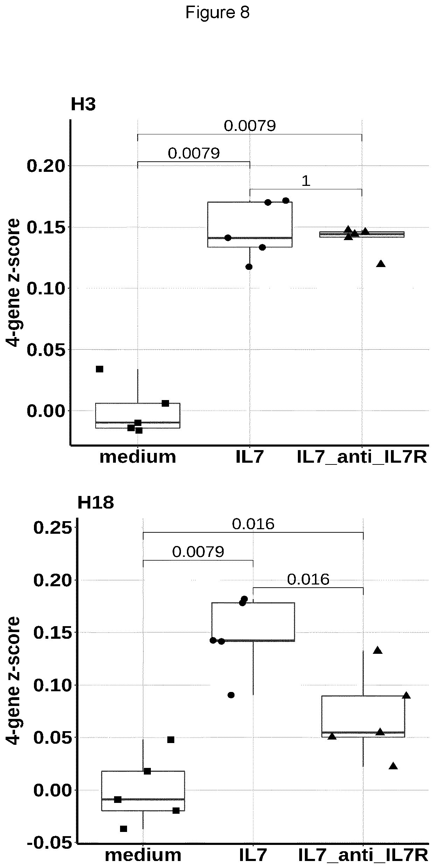

**Figure 8****:** shows the 4-gene signature z-score modulation (*IKZF4, KIAA0040, PGAP1 and SOS1)* after incubation with IL-7 and with an anti-IL-7R monoclonal antibody at 10 µg/mL, 30 minutes prior to IL-7 stimulation for 3 hours and 18 hours. The boxplot illustrates the differences in 4 genes z-score between medium and IL-7 stimulated PBMCs at 3 hours (H3) and 18 hours (H18) and in presence of an anti-IL-7R monoclonal antibody: medium condition (square); IL-7 stimulated condition (circle) and IL-7 stimulated condition with an IL-7R antagonist (N13B2-hVL6) (triangle).

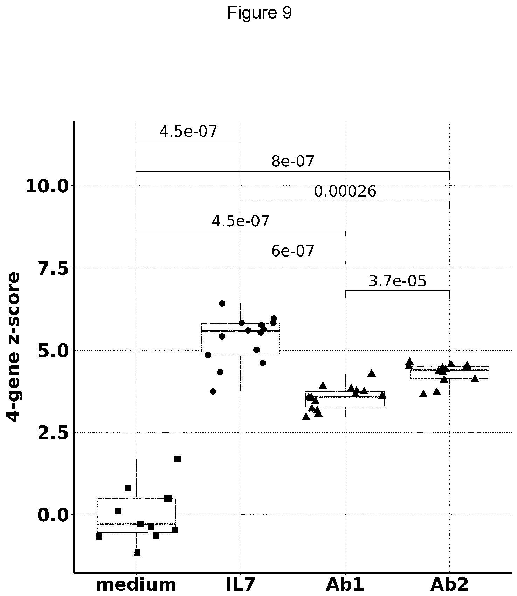

**Figure 9****:** shows the 4-gene signature z-score modulation *(IKZF4, KIAA0040, PGAP1 and SOS1)* determined by RT-qPCR after incubation with IL-7 and with two different anti-IL-7R monoclonal antibodies at 10 µg/mL, namely clones 1A11.H3L4 (indicated as Ab1) and clone HAL403a (indicated as Ab2), 30 minutes prior to IL-7 stimulation for 24 hours. The boxplot illustrates the differences in 4 genes z-score between medium and IL-7 stimulated PBMCs at 24 hours incubated with no antibody or in presence of each IL-7R antagonist: medium condition, IL-7 stimulated condition, IL-7 stimulated condition with IL-7R antagonist antibody Ab1 and IL-7 stimulated condition with IL-7R antagonist antibody Ab2. Statistical test: Wilcoxon.

### DETAILED DESCRIPTION OF THE INVENTION

The term "biomarker" refers to a distinctive indicator of a biological process, biological event, and/or pathologic condition. A biomarker can be analyzed at the nucleic acid or protein level; at the nucleic acid level, one can analyze the presence, absence, copy number of a gene, in wild type or mutant form, and/or its level of expression. At the protein level, one can analyze the level of expression and/or activity of a protein encoded by a genetic biomarker gene. In some embodiments, a biomarker may indicate a therapeutic outcome or likelihood thereof. Thus, a biomarker can be predictive, prognostic, or diagnostic and is therefore useful in methods of identifying (thereby diagnosing) or treating a patient as described herein. According to the present disclosure said biomarker may refer to a gene signature.

A "gene signature" is a single or combined group of genes in a cell with a uniquely characteristic pattern of gene expression. Clinical applications of gene signatures can be characterized as prognostic, diagnostic, and predictive signatures. In some embodiments, a prognostic gene signature can predict the likely outcome or course of a disease. Classifying a biological phenotype or medical condition based on a specific gene signature can serve as a prognostic biomarker for the associated phenotype or condition. In some embodiments, a diagnostic gene signature can serve as a biomarker that distinguishes phenotypically similar medical conditions that have a threshold of severity consisting of mild, moderate or severe phenotypes. In some embodiments, a predictive gene signature can predict the effect of treatment in patients or study participants that exhibit a particular disease phenotype. In some embodiments, these gene signatures can have implications in facilitating personalized medicine through identification of the most qualified subjects for optimal benefit of specific treatments. For example, gene signatures can be used to predict the survival or prognosis of a subject with a disease, to differentiate between different subtypes of a disease, to predict activation of a particular pathway or to select a group of patients for whom a particular treatment will be effective.

The term "subject" refers to an organism, typically a mammal, such as a rodent, a feline, a canine, and a primate. Preferably, a subject according to the invention is a human.

A "control subject" refers to a subject who has not been diagnosed as having a particular disease or disorder or pathological condition, such as an autoimmune disease, an inflammatory disease, a cancer disease or an infectious disease, and who does not suffer from any sign or symptom associated with said particular disease or disorder or pathological condition. Data obtained from samples from such control subject are referred to as reference data, e.g. "reference gene expression profile" standing for the gene expression profile obtained from control subjects.

The term "patient" refers to a subject who has been diagnosed as having an IL-7R disease or disorder or pathological condition, and who suffers from any sign or symptom associated with that disease. Data obtained from samples from such patient are referred to as "patient data", e.g. "gene expression profile of a patient" or "gene expression profile of a group of patients" standing for the gene expression profile obtained from respectively a patient or a group of patients.

A "biological sample" encompasses a variety of sample types obtained from an individual and that can be subjected to histological or cytological methods. The definition encompasses liquid samples, tissue samples or cellular samples of biological origin. The definition also includes samples that have been manipulated in any way after their procurement, such as by treatment with reagents, solubilization, or enrichment for certain components, such as polynucleotides. The term "biological sample" thus encompasses a clinical sample. The term "liquid sample" encompasses biological fluids such as whole blood, urine, saliva, cerebrospinal fluid, interstitial fluid, ocular fluid, synovial fluid, blood fractions such as plasma, serum or buffy coat. The term " tissue sample" encompasses solid tissue sample, tissue culture sample, biopsy sample, such as salivary gland biopsy, parotid gland biopsy, skin biopsy, synovial tissue biopsy or kidney biopsy. The term "cellular sample" encompasses cell in culture such as peripheral blood mononuclear cells, cell supernatant or cell lysate.

"IL-7R-mediated diseases" refers to diseases, disorders or conditions that are associated with a pathological increase or decrease of the IL-7R signaling pathway, and that can be treated, delayed or prevented, or whose symptoms or at least one of the symptoms can be stabilized, reduced or can disappear with an IL-7R modulator, such as an IL-7R antagonist (e.g. anti-IL-7R antagonist antibody or antigen binding fragment thereof) or an IL-7R agonist (e.g. IL-7). Such diseases include, but are not limited to, autoimmune diseases, inflammatory diseases, cancer diseases and infectious diseases, associated with a pathological increase or decrease of the IL-7R signaling pathway, and that can be treated, delayed or prevented, or whose symptoms or at least one of the symptoms can be stabilized, reduced or disappeared with an IL-7R modulator.

In a particular embodiment, said IL-7R-mediated disease is an inflammatory disease or an autoimmune disease, including inflammatory bowel disease (IBD), ulcerative colitis, irritable bowel syndrome, Crohn's disease, rheumatoid arthritis (RA), Sjögren's syndrome or Sjögren's disease, psoriasis, psoriatic arthritis, systemic lupus erythematosus (SLE), cutaneous lupus erythematosus (CLE), chronic discoid lupus erythematosus (CDLE), lupus nephritis, vasculitis, sepsis, systemic inflammatory response syndrome (SIRS), type I diabetes, Grave's disease, multiple sclerosis (MS), autoimmune myocarditis, Kawasaki disease, coronary artery disease, chronic obstructive pulmonary disease, interstitial lung disease, autoimmune thyroiditis, scleroderma, systemic sclerosis, osteoarthritis, atopic dermatitis, vitiligo, graft versus host disease, autoimmune nephritis, Goodpasture syndrome, chronic inflammatory demyelinating polyneuropathy, allergy, asthma, other autoimmune diseases that are a result of either acute or chronic inflammation. In a more particular embodiment, the inflammatory disease or autoimmune disease is inflammatory bowel disease (IBD), ulcerative colitis, irritable bowel syndrome, Crohn's disease, rheumatoid arthritis (RA), Sjogren's syndrome or Sjogren's disease, psoriasis, systemic lupus erythematosus (SLE), sepsis, systemic inflammatory response syndrome (SIRS), systemic sclerosis, multiple sclerosis, graft versus host disease, allergy or asthma. In a preferred embodiment, the inflammatory disease or autoimmune disease is Sjögren's syndrome or Sjögren's disease, systemic lupus erythematosus, rheumatoid arthritis, sclerosis, psoriasis, IBD or ulcerative colitis.

Sjogren's disease or syndrome is an autoimmune disease that is characterized by inflammatory manifestations and can be categorized as inflammatory disease. It often accompanies other immune system disorders presenting inflammatory manifestations such as rheumatoid arthritis, lupus, erythematosus, or progressive systemic sclerosis.

In a particular embodiment, said IL-7R-mediated disease is cancer disease such as leukemia, acute lymphoblastic leukemia, T-cell acute lymphoblastic leukemia, B-cell acute lymphoblastic leukemia, lymphocytic leukemia, lymphoma, Hodgkin's lymphoma, breast cancer, renal cancer, bladder cancer, lung cancer, pancreatic cancer, T cell cutaneous lymphoma. In a preferred embodiment, said cancer disease is leukemia or lymphoma.

In a particular embodiment, said IL-7R-mediated disease is a respiratory disease, such as chronic bronchitis, asthma including acute asthma and allergic asthma, cystic fibrosis, bronchiectasis, allergic or other rhinitis or sinusitis, coughs, pulmonary emphysema, pulmonary fibrosis or hyper-reactive airways, chronic obstructive pulmonary disease, and chronic obstructive lung disorder.

In a particular embodiment, said IL-7R-mediated disease is an infectious disease including but not limited to a bacterial infection, viral infection, yeast infection, Whipple's Disease, Prion Disease, methicillin-resistant staphylococcus aureus, hepatitis, syphilis, meningitis, malaria, tuberculosis, or influenza. In a more particular embodiment, the infectious disease is a viral infection, such as an infection caused by a virus such as HIV, HBV, HCV or coronavirus (e.g., SARS virus, MERS, virus, or COVID-19 virus).

The term "IL-7R signaling pathway" refers to the signaling pathway induced by the activation of IL-7R and/or the expression of IL-7R and/or the proliferation of IL-7R positive cells. The term "increase of the IL-7R signaling pathway" means a higher activation of IL-7R and/or a higher expression of IL-7R and/or a higher proliferation of IL-7R positive cells. The term "decrease of the IL-7R signaling pathway" means a lower activation of IL-7R and/or a lower expression of IL-7R and/or a lower proliferation of IL-7R positive cells.

The term "likely responder", or "likely responsive to a treatment" refers to a subject in whom the onset of at least one of the symptoms of the condition to be treated is delayed or prevented, upon or after treatment, or whose symptoms or at least one of the symptoms stabilize, diminish or disappear. According to the present disclosure, a patient is likely responsive to an IL-7R antagonist treatment when said antagonist has an effect of interfering with or inhibiting the IL-7R signaling pathway in said patient that can be correlated with therapeutic response to said antagonist treatment. In another particular embodiment, according to the present disclosure, a patient is likely responsive to an IL-7R agonist treatment when said agonist has an effect of inducing the IL-7R signaling pathway in said patient that can be correlated with therapeutic response to said agonist treatment.

"Therapeutic response" refers to the consequence of a medical treatment in a patient, the results of which are judged to be useful or favorable. For instance, a therapeutic response may be the delay or the prevention of at least one of the symptoms, upon or after treatment, or may be that the symptoms or at least one of the symptoms in patient stabilize, diminish or disappear.

According to the present disclosure, by "therapeutic response in a patient treated with an IL-7R modulator" or "IL-7R modulator therapeutic response", it is meant that the performed steps of IL-7R modulator administration, such as respectively an IL-7R antagonist or IL-7R agonist, result in improving the clinical condition of an animal or a human patient in need thereof, who suffers from disorder(s) associated respectively with an increase or decrease of the IL-7R signaling pathway. Such treatment aims at improving the clinical status of the animal or human patient, by eliminating or lowering the symptoms associated with the disorder(s) related to IL-7R signaling pathway.

In a particular embodiment, the therapeutic response in patient treated with an IL-7R antagonist refers to IL-7R antagonist administration that results in improving clinical condition of a patient who suffers from disorders associated with an increase of the IL-7R signaling pathway such as an autoimmune disease, an inflammatory disease, an allergic disease, a cancer disease, an infectious disease and a respiratory disease, in particular a cancer disease, an infectious disease, an autoimmune disease or an inflammatory disease.

In another particular embodiment, the therapeutic response in patient treated with an IL-7R agonist refers to IL-7R agonist administration that results in improving clinical condition of a patient who suffers from disorders associated with a decrease of the IL-7R signaling pathway such as an autoimmune disease, an inflammatory disease, an allergic disease, a cancer disease, an infectious disease and a respiratory disease, in particular a cancer disease, an infectious disease, an autoimmune disease or an inflammatory disease.

In the context of the present disclosure, the term "treating" or "treatment", as used herein, means reversing, alleviating, inhibiting the progress of, or preventing the disorder or condition to which such term applies, or reversing, alleviating, inhibiting the progress of, or preventing one or more symptoms of the disorder or condition to which such term applies.

According to the present disclosure, an "IL-7R antagonist or agonist property or effect" refers to a property or an effect of respectively decreasing or increasing the IL-7R signaling pathway. An antagonist effect encompasses the blocking, the antagonizing, the suppression or the reduction (to any degree including significantly) of the IL-7R signaling pathway.

### Gene signatures

The different methods according to the present disclosure involve determining the expression level of at least 2, 3 or 4 genes selected from the group consisting of: IKZF4, KIAA0040, PGAP1 and SOS1 genes in a patient sample. The different methods according to the present disclosure involves also determining the expression level of at least 2, 3, 4 or 5 genes selected from the group consisting of: IL-7R, PCED1B, TNFSF8, ADAM19 and MYBL1 genes in a patient sample.

The term "determining the gene expression profile of at least 2, 3 or 4 genes selected from the group consisting of: IKZF4, KIAA0040, PGAP1 and SOS1 genes" means that the gene expression profile of 2 of said genes, the gene expression profile of 3 of said genes or the gene expression profile of 4 of said genes is assessed. In a preferred embodiment, the gene expression profile of all 4 genes is assessed. The term " determining the gene expression profile of at least 2, 3, 4 or 5 genes selected from the group consisting of: IL-7R, PCED1B, TNFSF8, ADAM19 and MYBL1 genes" means that the gene expression profile of 2 of said genes, the gene expression profile of 3 of said genes, the gene expression profile of 4 of said genes or the gene expression profile of 5 of said genes is assessed. In a preferred embodiment, the expression level of all 5 genes is assessed.

The term "gene expression profile" refers to a pattern of expression of genes and may be determined by detecting the mRNA expression of said genes, analyzed a) either by calculating the Log2 Fold Change (Log2FC) in a biological sample for each gene selected from the group consisting of: IKZF4, KIAA0040, PGAP1 and SOS1 genes or for each gene selected from the group consisting of: IL-7R, PCED1B, TNFSF8, ADAM19 and MYBL1 genes, or b) by calculating a z-score "z" per sample of a patient and per gene as follows: z = (x-µ)/σ, where x is the value of the test sample, µ is the control population mean, and σ is the control population standard deviation. The z-score of 2 or more genes corresponds to the mean of the z-scores obtained for each gene individually. The 4-gene z-score of IKZF4, KIAA0040, PGAP1 and SOS1 genes is the mean of the z-scores obtained for each gene, i.e. the mean of {z-score of IKZF4 + z-score of KIAA0040 + z-score of PGAP1 + z-score of SOS1}. The 5-gene z-score of IL-7R, PCED1B, TNFSF8, ADAM19 and MYBL1 genes is the mean of the z-scores obtained for each gene, i.e. the mean of {z-score of IL-7R + z-score of PCED1B + z-score of TNFSF8 + z-score of ADAM19 + z-score of MYBL1}.

The gene expression profile is determined by using conventional techniques known to those of skill in the art such as RT-qPCR. RT-qPCR is a useful method already in place in most laboratories as it allows to quantify the expression of few genes rapidly and in a cost-effective manner, thus is well suited for a routinely detection or monitoring a drug or a clinical effect. Other conventional techniques known to those of skill in the art include, but are not limited to, techniques such as RNA-seq, qPCR, microarray analysis, serial analysis of gene expression (SAGE), MassARRAY technique, FISH, NanoString technique, high-coverage expression profiling (HiCEP), Next-Generation Sequencing (NGS) or a combination thereof.

As used herein, the term "reference gene expression profile" may refer to the gene expression profile determined in a biological sample of a control subject or in biological samples collected from a group of control subjects. The reference gene expression profile may alternatively be a predetermined value such as a threshold value, a cut-off value, a standard value or a range obtained from samples collected from a group of control subjects, that would be selected based upon the existing experimental and/or clinical conditions, as would be recognized by a person of ordinary skilled in the art.

Human *IKZF4* gene, also named Ikaros Family Zinc Finger 4 or Eos (Gene ID: 64375, updated on 23 June, 2022) encodes a transcription factor, expressed in lymphocytes and implicated in the control of lymphoid development. Human *IKZF4* gene encodes five variant transcripts which are referenced on NCBI as: transcript variant 1 NM_022465.4 of 5313 bp and transcript variant 2 NM_001351089.2 of 5384 bp, both updated on 16 April, 2022 and encoding zinc finger protein Eos isoform a (respectively NP_071910.3 and NP_001338018.1, both updated on 16 April, 2022), transcript variant 3 NM_001351090.1 of 5227 bp (updated on 17 April, 2022) encoding zinc finger protein Eos isoform b NP_001338019.1 of 540 amino acids updated on 17 April, 2022, transcript variant 4 NM_001351091.2 of 5042 bp (updated on 16 April, 2022) encoding zinc finger protein Eos isoform c NP_001338020.1 of 538 amino acids updated on 16 April, 2022 and transcript variant 5 NM_001351092.2 of 5219 bp (updated on 16 April, 2022) encoding zinc finger protein Eos isoform d NP_001338021.1 of 483 amino acids updated on 16 April, 2022.

Human *KIAA0040* gene (Gene ID: 9674, updated on 13 May, 2022) encodes an uncharacterized protein. Human *KIAA0040* gene encodes 6 transcripts variants which are referenced on NCBI as: transcript variant 1 NM_001162893.2 of 4616 bp, transcript variant 2 NM_014656.3 of 4548 bp, transcript variant 3 NM_001162894.2 of 4539 bp, transcript variant 4 NM_001162895.2 of 4474 bp, transcript variant 5 NM_001319230.2 of 4465 bp and transcript variant 6 NM_001319231.2 of 4623 bp, all being updated on 17 April, 2022. All six variants respectively encode the proteins referenced on NCBI as NP_001156365.1, NP_055471.2, NP_001156366.1, NP_001156367.1, NP_001306159.1 and NP_001306160.1, all corresponding to the same protein of 99 amino acids referenced Q15053 on Uniprot.

Human *PGAP1* gene, also named "post-GPI attachment to proteins inositol deacylase 1" or "GPI Inositol-Deacylase" (Gene ID: 80055, updated on 13 May, 2022) encodes a protein catalyzing the inositol deacylation of GPI (glycosyl-phosphatidyl-inositol), and required for the production of GPI that can attach to proteins, which may be an important factor in the transport of GPI-anchored proteins from the endoplasmic reticulum to the Golgi. Human *PGAP1* gene encodes three variant transcripts which are referenced on NCBI as: transcript variant 1 NM_024989.4 of 11090 bp (updated on 01 June, 2022), transcript variant 2 NM_001321099.2 of 11174 bp (updated on 05 June, 2022) and transcript variant 3 NM_001321100.2 of 11034 bp (updated on 05 June, 2022), encoding respectively different protein isoforms referenced NP_079265.2 (922 amino acids, updated on 01 June, 2022), NP_001308028.1 (748 amino acids, updated on 05 June, 2022) and NP_001308029.1 on NCBI (533 amino acids, updated on 05 June, 2022).

Human SOS1 gene, also named "SOS Ras/Rac guanine nucleotide exchange factor 1" or "Son of sevenless homolog 1" (Gene ID: 6654, updated on 12 June, 2022), encodes a protein that is a guanine nucleotide exchange factor for RAS proteins, membrane proteins that bind guanine nucleotides and participate in signal transduction pathways. Human SOS1 gene encodes three variant transcripts which are referenced on NCBI as: transcript variant 1 NM_024989.4 of 11090 bp (updated on 01 June, 2022), transcript variant 2 NM_001321099.2 of 11174 bp (updated on 05 June, 2022) and transcript variant 3 NM_001321100.2 of 11034 bp (updated on 05 June, 2022), encoding respectively different protein isoforms referenced on NCBI as NP_079265.2 (922 amino acids, updated on 01 June, 2022), NP_001308028.1 (748 amino acids, updated on 05 June, 2022) and NP_001308029.1 (533 amino acids, updated on 05 June, 2022).

Human *IL-7R* gene, also named "Interleukin-7 Receptor subunit alpha" or CD127 (Gene ID: 3575, updated on 24 Jul, 2022), encodes a receptor for interleukin-7 (IL-7) and for thymic stromal lymphopoietin (TSLP). Human IL-7R gene encodes the transcript variant NM_002185.5 of 4584 bp (NCBI reference, updated on 24 Jul, 2022), encoding the protein of 459 amino acids referenced NP_002176.2 (updated on 24 Jul, 2022).

Human *PCED18* gene, also named "PC-Esterase Domain Containing 1B" (Gene ID: 91523, updated on 13 May, 2022), encodes a protein that belongs to the GDSL/SGNH-like acyl-esterase family. Members of this family are hydrolases thought to function in modification of biopolymers on the cell surface. Human *PCED18* gene encodes two variant transcripts which are referenced on NCBI as: transcript variant 1 NM_138371.3 of 2310 bp (updated on 26 June, 2021) and transcript variant 2 NM_001281429.2 of 1842 bp (updated on 21 April, 2022), encoding for the same protein of 432 amino acids referenced Q96HM7 on Uniprot (and on NCBI as NP_612380.1 (updated on 26 June, 2021) and NP_001268358.1 (updated on 21 April, 2022)).

Human *TNFSF8* gene, also named "TNF Superfamily Member 8" (Gene ID: 944, updated on 13 May, 2022), encodes a cytokine that belongs to the tumor necrosis factor (TNF) ligand family and that is a ligand for TNFRSF8/CD30. Human *TNFSF8* gene encodes two variant transcripts which are referenced on NCBI as: transcript variant 1 NM_001244.4 of 3779 bp (updated on 08 June, 2022) and transcript variant 2 NM_001252290.1 of 1526 bp (updated on 17 April, 2022), encoding respectively different protein isoforms referenced on NCBI as NP_001235.1 (234 amino acids, updated on 08 June, 2022) and NP_001239219.1 (164 amino acids, updated on 17 April, 2022).

Human *ADAM19* gene, also named "ADAM Metallopeptidase Domain 19" (Gene ID: 8728, updated on 13 May, 2022), encodes a member of the ADAM (a disintegrin and metalloprotease domain) family. Human *ADAM19* gene encodes the transcript referenced NM_033274.5 on NCBI (6481 bp, updated on 06 June, 2022) which encodes the protein referenced NP_150377.1 on NCBI (918 amino acids, updated on 06 June, 2022).

Human *MYBL1* gene, also named "MYB Proto-Oncogene Like 1" (Gene ID: 4603, updated on 23 May, 2022), encodes a protein that enables DNA-binding transcription activator activity, RNA polymerase II-specific and RNA polymerase II cis-regulatory region sequence-specific DNA binding activity and that is involved in positive regulation of transcription by RNA polymerase II. Human *MYBL1* gene encodes three variant transcripts referenced on NCBI as: transcript variant 1 NM_001080416.4 of 5161 bp (updated on 06 June, 2022), transcript variant 2 NM_001144155.3 of 4981 bp (updated on 17 April, 2022) and transcript variant 3 NM_001294282.2 of 4978 bp (updated on 18 April, 2022), encoding respectively different protein isoforms referenced on NCBI as NP_001073885.1 (752 amino acids, updated on 06 June, 2022), NP_001138227.1 (692 amino acids, updated on 17 April, 2022) and NP_001281211.1 (691 amino acids, updated on 18 April, 2022).

### Method of identification of a patient likely suffering from an IL-7R-mediated disease

The expression profile of at least 2, 3, 4 or 5 genes, in particular the expression profile of 4 or 5 genes, is informative to identify a patient likely suffering from a disease associated with an increase or decrease of the IL-7R signaling pathway.

The biomarkers according to the present disclosure make it possible to identify the patient as suffering from a disease associated with an increase or decrease of the IL-7R signaling pathway by determining the gene expression profile of at least 2, 3 or 4 genes selected from the group consisting of: IKZF4, KIAA0040, PGAP1 and SOS1 genes in a patient sample, or by determining the gene expression profile of at least 2, 3, 4 or 5 genes selected from the group consisting of: IL-7R, PCED1B, TNFSF8, ADAM19 and MYBL1 genes in a patient sample, and by comparing whether said determined gene expression profile of said genes is higher or lower compared to a reference gene expression profile obtained in biological samples collected from a group of control subjects.

The present invention is directed to a method of identification of a patient likely suffering from a disease associated with an increase or decrease of the IL-7R signaling pathway comprising the steps of:
a) determining the gene expression profile in a biological sample of said patient of at least 2, 3 or 4 genes selected from the group consisting of: IKZF4, KIAA0040, PGAP1 and SOS1 genes, or
b) determining the gene expression profile in a biological sample of said patient of at least 2, 3, 4 or 5 genes selected from the group consisting of: IL-7R, PCED1B, TNFSF8, ADAM19 and MYBL1 genes, and
c) comparing the gene expression profile determined in a) or b) to a reference gene expression profile,
wherein respectively a higher or lower gene expression profile of said genes in said patient sample as compared to the reference gene expression profile of said genes is indicative that the patient is likely suffering from a disease associated respectively with an increase or decrease of the IL-7R signaling pathway.

In a particular embodiment, the present invention relates to a method of identification of a patient likely suffering from a disease associated with an increase of the IL-7R signaling pathway comprising the steps of determining the gene expression profile in a biological sample of said patient of at least 2, 3 or 4 genes selected from the group consisting of: IKZF4, KIAA0040, PGAP1 and SOS1 genes, and comparing said determined gene expression profile to a reference gene expression profile of said genes, wherein a higher gene expression profile of said genes in said patient sample as compared to the reference gene expression profile of said genes is indicative that the patient is likely suffering from a disease associated with an increase of the IL-7R signaling pathway.

The present invention also relates to a method of identification of a patient likely suffering from a disease associated with a decrease of the IL-7R signaling pathway comprising the steps of determining the gene expression profile in a biological sample of said patient of at least 2, 3 or 4 genes selected from the group consisting of: IKZF4, KIAA0040, PGAP1 and SOS1 genes, and comparing said determined gene expression profile to a reference gene expression profile of said genes, wherein a lower gene expression profile of said genes in said patient sample as compared to the reference gene expression profile of said genes is indicative that the patient is likely suffering from a disease associated with a decrease of the IL-7R signaling pathway.

In a particular embodiment, the present invention relates to a method of identification of a patient likely suffering from a disease associated with an increase of the IL-7R signaling pathway comprising the steps of determining the gene expression profile in a biological sample of said patient of at least 2, 3, 4 or 5 genes selected from the group consisting of: IL-7R, PCED1B, TNFSF8, ADAM19 and MYBL1 genes, and comparing said determined gene expression profile to a reference gene expression profile of said genes, wherein a higher gene expression profile of said genes in said patient sample as compared to the reference gene expression profile of said genes is indicative that the patient is likely suffering from a disease associated with an increase of the IL-7R signaling pathway.

The present invention also relates to a method of identification of a patient likely suffering from a disease associated with a decrease of the IL-7R signaling pathway comprising the steps of determining the gene expression profile in a biological sample of said patient of at least 2, 3, 4 or 5 genes selected from the group consisting of: IL-7R, PCED1B, TNFSF8, ADAM19 and MYBL1 genes, and comparing said determined gene expression profile to a reference gene expression profile of said genes, wherein a lower gene expression profile of said genes in said patient sample as compared to the reference gene expression profile of said genes is indicative that the patient is likely suffering from a disease associated with a decrease of the IL-7R signaling pathway.

In a more particular embodiment, the present invention relates to said method of identification of a patient likely suffering from a disease associated with a an increase or decrease of the IL-7R signaling pathway, comprising the step of determining the gene expression profile in a biological sample of said patient of at least 2, 3, 4 or 5 genes, wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells.

In a particular embodiment, said method of identification of a patient likely suffering from a disease associated with an increase or decrease of the IL-7R signaling pathway, comprises the step of determining the gene expression profile by using conventional techniques known to those of skill in the art such as RT-qPCR, RNA-seq, qPCR, microarray analysis, serial analysis of gene expression (SAGE), MassARRAY technique, FISH, NanoString technique, high-coverage expression profiling (HiCEP), Next-Generation Sequencing (NGS) or a combination thereof.

As used herein, "a disease associated with an increase or decrease of the IL-7R signaling pathway" refers to an IL-7R-mediated disease, i.e. to disease, disorder or condition that is associated with a pathological increase or decrease of the IL-7R signaling pathway. Such diseases include, but are not limited to, autoimmune diseases, inflammatory diseases, cancer diseases and infectious diseases. In a particular embodiment, said disease associated with an increase or decrease of the IL-7R signaling pathway is an autoimmune disease, an inflammatory disease, a cancer disease or an infectious disease. In a more particular embodiment, said disease associated with an increase or decrease of the IL-7R signaling pathway is an autoimmune disease or inflammatory disease such as Sjogren's syndrome or Sjogren's disease, systemic lupus erythematosus, rheumatoid arthritis, sclerosis, psoriasis, intestinal bowel disease or ulcerative colitis. In another more particular embodiment, said disease associated with an increase or decrease of the IL-7R signaling pathway is a cancer disease such as T-cell acute lymphoblastic leukemia, B-cell acute lymphoblastic leukemia, chronic lymphocytic leukemia, T-cell prolymphocytic leukemia and Hodgkin's lymphoma.

### Method of identification of a patient likely responsive to treatment with an IL-7R modulator

The expression profile of at least 2, 3, 4 or 5 genes, in particular the expression profile of 4 or 5 genes, is informative to identify a patient likely suffering from a disease associated with a pathological increase or decrease of the IL-7R signaling pathway who would thus be likely responsive to treatment with an IL-7R modulator, respectively with an IL-7R antagonist or an IL-7R agonist.

The biomarkers according to the present disclosure make it possible to identify a patient as likely responsive to treatment with an IL-7R modulator by determining the gene expression profile of at least 2, 3 or 4 genes selected from the group consisting of: IKZF4, KIAA0040, PGAP1 and SOS1 genes in a biological sample of said patient, or by determining the gene expression profile of at least 2, 3, 4 or 5 genes selected from the group consisting of: IL-7R, PCED1B, TNFSF8, ADAM19 and MYBL1 genes in a biological sample of said patient, and by comparing whether said determined gene expression profile is higher or lower compared to a reference gene expression profile.

The present invention is directed to a method of identification of a patient likely responsive to treatment with an IL-7R modulator, i.e IL-7R agonist or IL-7R antagonist, prior to said treatment, by:
a) determining in a biological sample of said patient, whether the patient's gene expression profile of at least 2, 3 or 4 genes selected from the group consisting of: IKZF4, KIAA0040, PGAP1 and SOS1 genes is higher or lower compared to a reference gene expression profile, or
b) determining in a biological sample of said patient, whether the patient's gene expression profile of at least 2, 3, 4 or 5 genes selected from the group consisting of: IL-7R, PCED1B, TNFSF8, ADAM19 and MYBL1 genes is higher or lower compared to a reference gene expression profile,
wherein a higher or lower gene expression profile determined in a) or b) is indicative that the patient is likely responsive to treatment with an IL-7R antagonist or with an IL-7R agonist respectively.

In a particular embodiment, the present invention is directed to a method of identification of a patient likely responsive to treatment with an IL-7R agonist, prior to said treatment, by determining in a biological sample of said patient, whether the patient's gene expression profile of at least 2, 3 or 4 genes selected from the group consisting of: IKZF4, KIAA0040, PGAP1 and SOS1 genes is higher or lower compared to a reference gene expression profile, wherein a lower gene expression profile is indicative that the patient is likely responsive to treatment with an IL-7R agonist.

In another particular embodiment, the present invention is directed to a method of identification of a patient likely responsive to treatment with an IL-7R antagonist, prior to said treatment, by determining in a biological sample of said patient whether the patient's gene expression profile of at least 2, 3 or 4 genes selected from the group consisting of: IKZF4, KIAA0040, PGAP1 and SOS1 genes is higher or lower compared to a reference gene expression profile, wherein a higher gene expression profile is indicative that the patient is likely responsive to treatment with an IL-7R antagonist.

In a particular embodiment, the present invention is directed to a method of identification of a patient likely responsive to treatment with IL-7R agonist, prior to said treatment, by determining in a biological sample of said patient, whether the patient's gene expression profile of at least 2, 3, 4 or 5 genes selected from the group consisting of: IL-7R, PCED1B, TNFSF8, ADAM19 and MYBL1 genes is higher or lower compared to a reference gene expression profile, wherein a lower gene expression profile is indicative that the patient is likely responsive to treatment with an IL-7R agonist.

In another particular embodiment, the present invention is directed to a method of identification of a patient likely responsive to treatment with IL-7R antagonist, prior to said treatment, by determining in a biological sample of said patient, whether the patient's gene expression profile of at least 2, 3, 4 or 5 genes selected from the group consisting of: IL-7R, PCED1B, TNFSF8, ADAM19 and MYBL1 genes is higher or lower compared to a reference gene expression profile, wherein a higher gene expression profile is indicative that the patient is likely responsive to treatment with an IL-7R antagonist.

In a more particular embodiment, the present invention relates to said method of identification of a patient likely responsive to treatment with an IL-7R modulator, i.e IL-7R agonist or IL-7R antagonist, prior to said treatment, by determining in a biological sample of said patient the patient's gene expression profile of at least 2, 3, 4 or 5 genes selected from the group consisting of: IL-7R, PCED1B, TNFSF8, ADAM19 and MYBL1 genes, wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells.

In a particular embodiment, said method of identification of a patient likely responsive to treatment with an IL-7R modulator, i.e IL-7R agonist or IL-7R antagonist, prior to said treatment comprises the step of determining in a biological sample of said patient the patient's gene expression profile by using conventional techniques known to those of skill in the art such as RT-qPCR, RNA-seq, qPCR, microarray analysis, serial analysis of gene expression (SAGE), MassARRAY technique, FISH, NanoString technique, high-coverage expression profiling (HiCEP), Next-Generation Sequencing (NGS) or a combination thereof.

### Method for evaluating the therapeutic response of a patient treated with an IL-7R modulator

The expression profile of at least 2, 3, 4 or 5 genes, in particular the expression profile of 4 or 5 genes, is also informative for evaluating the therapeutic response to an IL-7R modulator (i.e. an IL-7R agonist or an IL-7R antagonist) of a patient treated with said IL-7R modulator.

The biomarkers according to the present disclosure make it possible to evaluate the therapeutic response to an IL-7R modulator of a patient treated with said IL-7R modulator, by determining the gene expression profile of at least 2, 3 or 4 genes selected from the group consisting of: IKZF4, KIAA0040, PGAP1 and SOS1 genes in a biological sample of said patient, or by determining the gene expression profile of at least 2, 3, 4 or 5 genes selected from the group consisting of: IL-7R, PCED1B, TNFSF8, ADAM19 and MYBL1 genes in a biological sample of said patient, and by comparing whether said determined gene expression profile of said genes is higher or lower compared to the gene expression profile determined in the same patient before said treatment or earlier during said treatment.

The biomarkers according to the present disclosure make it also possible to evaluate the therapeutic response to an IL-7R modulator of a patient treated with said IL-7R modulator, by determining the gene expression profile of at least 2, 3 or 4 genes selected from the group consisting of: IKZF4, KIAA0040, PGAP1 and SOS1 genes in a biological sample of said patient, or by determining the gene expression profile of at least 2, 3, 4 or 5 genes selected from the group consisting of: IL-7R, PCED1B, TNFSF8, ADAM19 and MYBL1 genes in a biological sample of said patient, and by comparing whether said determined gene expression profile of said genes is unchanged compared to the gene expression profile determined in the same patient before said treatment or earlier during said treatment.

In a particular embodiment, the present invention is directed to a method for evaluating the therapeutic response to an IL-7R agonist of a patient treated with said IL-7R agonist, comprising the steps of determining in a biological sample of said patient, the gene expression profile of at least 2, 3 or 4 genes selected from the group consisting of: IKZF4, KIAA0040, PGAP1 and SOS1 genes, and comparing said gene expression profile to a gene expression profile determined in the same patient before said treatment or earlier during said treatment, wherein a higher gene expression profile is indicative that the patient is likely responsive to the treatment with the IL-7R agonist and wherein a lower gene expression profile is indicative that the patient is likely not responsive to the treatment with the IL-7R agonist.

In another particular embodiment, the present invention is directed to a method for evaluating the therapeutic response to an IL-7R antagonist of a patient treated with said IL-7R antagonist, comprising the steps of determining in a biological sample of said patient, the patient's gene expression profile of at least 2, 3 or 4 genes selected from the group consisting of: IKZF4, KIAA0040, PGAP1 and SOS1 genes, and, comparing said gene expression profile to a gene expression profile determined in said patient before said treatment or earlier during said treatment, wherein a lower gene expression profile is indicative that the patient is likely responsive to the treatment with the IL-7R antagonist and wherein a higher gene expression profile is indicative that the patient is likely not responsive to the treatment with the IL-7R antagonist.

In a more particular embodiment, the present invention relates to said method for evaluating the therapeutic response to an IL-7R modulator of a patient treated with said IL-7R modulator, by determining in a biological sample of said patient the patient's gene expression profile of at least 2, 3 or 4 genes selected from the group consisting of: IKZF4, KIAA0040, PGAP1 and SOS1 genes, wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells.

In a particular embodiment, the invention is directed to a method for evaluating the therapeutic response to an IL-7R agonist of a patient treated with said IL-7R agonist, comprising the steps of determining in a biological sample of said patient, the patient's gene expression profile of at least 2, 3, 4 or 5 genes selected from the group consisting of: IL-7R, PCED1B, TNFSF8, ADAM19 and MYBL1 genes, and, comparing said gene expression profile to a gene expression profile determined in the patient before said treatment or earlier during said treatment, wherein a higher gene expression profile is indicative that the patient is likely responsive to the treatment with the IL-7R agonist and wherein a lower gene expression profile is indicative that the patient is likely not responsive to the treatment with the IL-7R agonist.

In another particular embodiment, the present invention is directed to a method for evaluating the therapeutic response to an IL-7R antagonist of a patient treated with said IL-7R antagonist comprising the steps of determining in a biological sample of said patient, the patient's gene expression profile of at least 2, 3, 4 or 5 genes selected from the group consisting of: IL-7R, PCED1B, TNFSF8, ADAM19 and MYBL1 genes, and, comparing said gene expression profile to a gene expression profile determined in the patient before said treatment or earlier during said treatment, wherein a lower gene expression profile is indicative that the patient is likely responsive to the treatment with the IL-7R antagonist and wherein a higher gene expression profile is indicative that the patient is likely not responsive to the treatment with the IL-7R antagonist.

In a more particular embodiment, the present invention relates to said method for evaluating the therapeutic response to an IL-7R modulator of a patient treated with said IL-7R modulator, by determining in a biological sample of said patient the patient's gene expression profile of at least 2, 3, 4 or 5 genes selected from the group consisting of: IL-7R, PCED1B, TNFSF8, ADAM19 and MYBL1 genes, wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells.

In a particular embodiment, the invention is directed to a method for evaluating the therapeutic response to an IL-7R modulator (i.e. an IL-7R agonist or an IL-7R antagonist) of a patient treated with said IL-7R modulator, by determining the gene expression profile of at least 2, 3 or 4 genes selected from the group consisting of: IKZF4, KIAA0040, PGAP1 and SOS1 genes in a biological sample of said patient, or by determining the gene expression profile of at least 2, 3, 4 or 5 genes selected from the group consisting of: IL-7R, PCED1B, TNFSF8, ADAM19 and MYBL1 genes in a biological sample of said patient, and, comparing said gene expression profile to a gene expression profile determined in the patient before said treatment or earlier during said treatment, wherein an unchanged gene expression profile is indicative that the patient is likely not responsive to the treatment with the IL-7R modulator.

In a more particular embodiment, the present invention relates to a method for evaluating the therapeutic response to an IL-7R modulator of a patient treated with said IL-7R modulator, by determining in a biological sample of said patient the patient's gene expression profile, wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells.

In a particular embodiment, the gene expression profile of said biomarkers is evaluated in a biological sample collected from a patient before treatment with an IL-7R modulator and/or 1, 2, 3, 7, 10, 14, 21, 28, 30, 35, 40, 45, 50, 55, 60, 70, 80, 90, 100, 120, 150, 180, 210, 250 or 270 days and/or 1, 2, 3, 4, 5, 6, 9 or 12 months after a dose administration of IL-7R modulator, i.e. an IL-7R agonist or an IL-7R antagonist.

In a particular embodiment, said method for evaluating the therapeutic response to an IL-7R modulator (i.e. an IL-7R agonist or an IL-7R antagonist) of a patient treated with said IL-7R modulator, by determining cited gene expression profile, comprises the step of determining gene expression profile by detecting the mRNA expression of said genes, using conventional techniques known to those of skill in the art such as RT-qPCR, RNA-seq, qPCR, microarray analysis, serial analysis of gene expression (SAGE), MassARRAY technique, FISH, NanoString technique, high-coverage expression profiling (HiCEP), Next-Generation Sequencing (NGS) or a combination thereof.

### Method for predicting the disease severity in a patient suffering from an IL-7R-mediated disease

In a particular embodiment, the present invention is directed to a method for predicting an IL-7R-mediated disease severity in a patient suffering from said disease, comprising the steps of determining in a biological sample of said patient the gene expression profile of at least 2, 3 or 4 genes selected from the group consisting of: IKZF4, KIAA0040, PGAP1 and SOS1 genes, and, comparing the determined gene expression profile to a gene expression profile of a representative group of patients suffering from said IL-7R-mediated disease, and wherein the patient's gene expression profile is indicative of a high disease activity when the patient's gene expression profile is significantly higher than the gene expression profile of said representative group of patients.

In a more particular embodiment, the present invention is directed to a method for predicting an IL-7R-mediated disease severity in a patient suffering from said disease, comprising the steps of determining in a biological sample of said patient the gene expression profile of at least 2, 3 or 4 genes selected from the group consisting of: IKZF4, KIAA0040, PGAP1 and SOS1 genes, and, comparing the determined gene expression profile to a gene expression profile of a representative group of patients suffering from said IL-7R-mediated disease, and wherein the patient's gene expression profile is indicative of a low disease activity when the patient's gene expression profile is significantly lower than the gene expression profile of said representative group of patients.

In a particular embodiment, the present invention is directed to a method for predicting an IL-7R-mediated disease severity in a patient suffering from said disease, comprising the steps of determining in a biological sample of said patient the gene expression profile of at least 2, 3, 4 or 5 genes selected from the group consisting of: IL-7R, PCED1B, TNFSF8, ADAM19 and MYBL1 genes, and, comparing the determined gene expression profile to a gene expression profile of a representative group of patients suffering from said IL-7R-mediated disease, and wherein the patient's gene expression profile is indicative of a high disease activity when the patient's gene expression profile is significantly higher than the gene expression profile of said representative group of patients.

In a more particular embodiment, the present invention is directed to a method for predicting an IL-7R-mediated disease severity in a patient suffering from said disease, comprising the steps of determining in a biological sample of said patient the gene expression profile of at least 2, 3, 4 or 5 genes selected from the group consisting of: IL-7R, PCED1B, TNFSF8, ADAM19 and MYBL1 genes, and, comparing the determined gene expression profile to a gene expression profile of a representative group of patients suffering from said IL-7R-mediated disease, and wherein the patient's gene expression profile is indicative of a low disease activity when the patient's gene expression profile is significantly lower than the gene expression profile of said representative group of patients.

In a more particular embodiment, the invention is directed to a method for predicting the disease severity in a patient suffering from an IL-7R-mediated disease, wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells.

In a more particular embodiment, said method for predicting the disease severity in a patient suffering from an IL-7R-mediated disease comprises the step of determining gene expression profile, by using conventional techniques known to those of skill in the art such as RT-qPCR,RNA-seq, qPCR, microarray analysis, serial analysis of gene expression (SAGE), MassARRAY technique, FISH, NanoString technique, high-coverage expression profiling (HiCEP), Next-Generation Sequencing (NGS) or a combination thereof.

In a more particular embodiment, the invention is directed to a method for predicting the disease activity in a patient suffering from an IL-7R-mediated disease, wherein said disease activity is correlated with a marker of an IL-7R-mediated disease. Said marker of an IL-7R-mediated disease is rheumatoid factor (RF), anti-cyclic citrullinated antibody (anti-CCP), antinuclear autoantibodies (ANA), anti-double-stranded DNA antibodies, antibodies to extractable nuclear antigens (ENA), antineutrophil cytoplasmic autoantibodies (ANCA) and/or said marker of an IL-7R-mediated disease is autoantibodies to Sjögren Syndrom Antigen A (SSA or Ro) 60 kDa (Ro/SSA 60), SSA 52 kDa (or Ro/SSA52), Sjögren Syndrom Antigen B (SSB or La), Jo-I, Smith antigen (Sm or SM), ribonucleoprotein (U1.RNP), topoisomerase (Scl-70) and centromer B peptide (CENB-P), and/or said marker of an IL-7R-mediated disease is the presence and/or the proportion of lesional tissue, the presence and/or the proportion of lymphocytic infiltrates in tissues or a specific criteria comprised in a clinical scale such as EULAR Sjögren's syndrome disease activity index (ESSDAI) scale.

In a more particular embodiment, the present invention is directed to said method for predicting the disease activity in a patient suffering from an IL-7R-mediated disease, wherein said IL-7R-mediated disease is preferably an autoimmune disease, an inflammatory disease, a cancer or an infectious disease, more preferably wherein the autoimmune disease or inflammatory disease is Sjogren's syndrome or Sjogren's disease, systemic lupus erythematosus, rheumatoid arthritis, sclerosis, psoriasis, intestinal bowel disease or ulcerative colitis, more preferably wherein the cancer disease is T-cell acute lymphoblastic leukemia, B-cell acute lymphoblastic leukemia, chronic lymphocytic leukemia, T-cell prolymphocytic leukemia and Hodgkin's lymphoma.

### Method for selecting a compound likely effective in the treatment of an IL-7R-mediated disease

The present invention is directed to a method for selecting a compound likely effective in the treatment of a disease associated with an increase of the IL-7R signaling pathway comprising the steps of:
a) culturing cells from a biological sample,
b) determining in said cell culture, the gene expression profile of at least 2, 3 or 4 genes selected from the group consisting of: IKZF4, KIAA0040, PGAP1 and SOS1 genes,
c) comparing the determined gene expression profile to a reference gene expression profile, and
d) selecting a compound that induces a lower gene expression profile of said genes in said cell culture as compared to a reference gene expression profile.

The present invention is also directed to a method for selecting a compound likely effective in the treatment of a disease associated with a decrease of the IL-7R signaling pathway comprising the steps of:
a) culturing cells from a biological sample, such as cells isolated from blood samples or tissue samples,
b) determining in said cell culture, the gene expression profile of at least 2, 3 or 4 genes selected from the group consisting of: IKZF4, KIAA0040, PGAP1 and SOS1 genes,
c) comparing the determined gene expression profile to a reference gene expression profile, and
d) selecting a compound that induces a higher gene expression profile of said genes in said cell culture as compared to a reference gene expression profile.

In another particular embodiment, the present invention is directed to a method for selecting a compound likely effective in the treatment of a disease associated with an increase of the IL-7R signaling pathway comprising the steps of:
a) culturing cells from a biological sample, such as cells isolated from blood samples or tissue samples,
b) determining in said cell culture, the gene expression profile of at least 2, 3, 4 or 5 genes selected from the group consisting of: IL-7R, PCED1B, TNFSF8, ADAM19 and MYBL1 genes,
c) comparing the determined gene expression profile to a reference gene expression profile, and
d) selecting a compound that induces a lower gene expression profile of said genes in said cell culture as compared to a reference gene expression profile.

In another particular embodiment, the present invention is directed to a method for selecting a compound likely effective in the treatment of a disease associated with a decrease of the IL-7R signaling pathway comprising the steps of:
a) culturing cells from a biological sample, such as cells isolated from blood samples or tissue samples,
b) determining in said cell culture, the gene expression profile of at least 2, 3, 4 or 5 genes selected from the group consisting of: IL-7R, PCED1B, TNFSF8, ADAM19 and MYBL1 genes,
c) comparing the determined gene expression profile to a reference gene expression profile, and
d) selecting a compound that induces a higher gene expression profile of said genes in said cell culture as compared to a reference gene expression profile.

In a particular embodiment, said method for selecting a compound likely effective in the treatment of a disease associated with an increase of the IL-7R signaling pathway results in the selection of a compound that induces a lower gene expression profile as compared to a reference gene expression profile, wherein said compound inducing a lower gene expression profile is an IL-7R antagonist.

In a particular embodiment, said method for selecting a compound likely effective in the treatment of a disease associated with a decrease of the IL-7R signaling pathway results in the selection of a compound that induces a higher gene expression profile as compared to a reference gene expression profile, wherein said compound inducing a higher gene expression profile is an IL-7R agonist.

In a particular embodiment, said method for selecting a compound likely effective in the treatment of a disease associated with an increase or decrease of the IL-7R signaling pathway comprises the step of culturing cells from a biological sample, wherein said biological sample is a liquid sample, in particular whole blood sample or synovial fluid, or a tissue sample, in particular salivary gland biopsy, parotid gland biopsy, skin biopsy, synovial tissue biopsy or kidney biopsy, or a cell sample, in particular peripheral blood mononuclear cells.

In a more particular embodiment, said method for selecting a compound likely effective in the treatment of a disease associated with an increase or decrease of the IL-7R signaling pathway comprises the step of determining gene expression profile, wherein gene expression profile is determined by detecting the mRNA expression of said genes, using conventional techniques known to those of skill in the art such as RT-qPCR. RT-qPCR is a useful method already in place in most laboratories as it allows to quantify the expression of few genes rapidly and in a cost-effective manner, thus is well suited for a routinely detection or monitoring a drug or a clinical effect, for instance. Other conventional techniques known to those of skill in the art include, but are not limited to, techniques such as RNA-seq, qPCR, microarray analysis, serial analysis of gene expression (SAGE), MassARRAY technique, FISH, NanoString technique, high-coverage expression profiling (HiCEP), Next-Generation Sequencing (NGS) or a combination thereof.

### Kit

In one embodiment, the present invention relates to a kit comprising a set of reagents that specifically detects the gene expression profile of at least 2, 3 or 4 genes selected from the group consisting of: IKZF4, KIAA0040, PGAP1 and SOS1 genes, and/or the gene expression profile of at least 2, 3, 4 or 5 genes selected from the group consisting of: IL-7R, PCED1B, TNFSF8, ADAM19, MYBL1 genes, wherein said reagents are primer pairs and/or probes specific of each gene.

Preferably, the kit comprises containers each comprising one or more compounds at a concentration or in an amount that facilitates the reconstitution and/or the use of a set of reagents that specifically detects the gene expression profile of at least 2, 3 or 4 genes selected from the group consisting of: IKZF4, KIAA0040, PGAP1 and SOS1 genes, and/or the gene expression profile of at least 2, 3, 4 or 5 genes selected from the group consisting of: IL-7R, PCED1B, TNFSF8, ADAM19, MYBL1 genes and the implementation of the method according to the disclosure. For instance, the kit may include one or more articles and/or reagents for performance of the method, such as buffer solutions, enzymes such as deoxynucleotides (dNTPs), Taq-polymerase and reverse transcriptase, DNase, RNase inhibitor DEPC-water, sterile water and/or means for obtaining the test sample itself, e.g. means for obtaining and/or isolating a sample. The kit allows the determination of mRNA expression by conventional techniques known to those of skill in the art, such as RNA-seq, RT-qPCR, qPCR, microarray analysis, serial analysis of gene expression (SAGE), MassARRAY technique, FISH, NanoString technique, high-coverage expression profiling (HiCEP), Next-Generation Sequencing (NGS) or a combination thereof.

In particular, the kit contains primer pair and/or probes specific of at least 2, 3 or 4 genes selected from the group consisting of: IKZF4, KIAA0040, PGAP1 and SOS1 genes, and/or the gene expression profile of at least 2, 3, 4 or 5 genes selected from the group consisting of: IL-7R, PCED1B, TNFSF8, ADAM19, MYBL1 genes. The design of suitable primers and/or probes is routine and well within the capabilities of the skilled person.

The kit may also comprise instructions indicating the methods for preparing and/or using the reagents to determine the expression level of said genes according to the methods of the disclosure.

The present invention also relates to the use of the kit for a method according to the present disclosure.

### IL-7R modulator

The term "IL-7R modulator" refers to a compound that has IL-7R antagonist or agonist properties and that modulates (respectively decreases or increases) the IL-7R signaling pathway, i.e. IL-7R expression, IL-7R activity and/or the proliferation of IL-7R positive cells.

In a preferred embodiment, said IL-7R modulator (e.g. IL-7R antagonist or IL-7R agonist) can be selected from the group consisting of: protein, polypeptide or peptide modulator such as components of the IL-7R signaling pathway (e.g. IL-7), variants or derivative thereof (fragments, fusion proteins, soluble proteins, dominant negative mutants), antibodies directed to component of IL-7R signaling pathway including fragment and expressible derivative thereof and antigen-binding antibody mimetics. In a particular embodiment, said IL-7R modulator may be an IL-7R antagonist or agonist antibody or antigen-binding fragment thereof, preferably a monoclonal antibody.

The term "antibody" (Ab) or "immunoglobulin" (Ig), as used herein, refers toa tetramer comprising two heavy (H) chains (about 50-70 kDa) and two light (L) chains (about 25 kDa) inter-connected by disulfide bonds. Each heavy chain is comprised of a heavy chain variable domain (VH) and a heavy chain constant region (CH). Each light chain is composed of a light chain variable domain (VL) and a light chain constant region (CL). The VH and VL domains can be subdivided further into regions of hypervariability, termed "complementarity determining regions" (CDRs), interspersed with regions that are more conserved, termed "framework regions" (FRs). Each VH and VL is composed of three CDRs (H-CDR herein designates a CDR from the heavy chain; and L-CDR herein designates a CDR from the light chain) and four FRs, arranged from amino-terminus to carboxyl-terminus in the following order: FR1, CDR1, FR2, CDR2, FR3, CDR3, FR4. The assignment of amino acid numbers in the heavy or light chain may be in accordance with EU numbering or IMGT^{®} definitions (Lefranc et al., Dev Comp Immunol 27(1):55-77 (2003)); or the definitions of Kabat, Sequences of Proteins of Immunological Interest (National Institutes of Health, Bethesda, MD (1987 and 1991)); Chothia & Lesk, J. Mol. Biol. 196:901-91730 (1987); or Chothia et al., Nature 342:878-883 (1989). The term "monoclonal antibody" as used herein refers to an antibody that displays a single binding specificity and affinity for a particular epitope.

The term "antigen-binding fragment" of an antibody (or simply "antibody fragment"), as used herein, refers to one or more fragments of an antibody that retain the ability to specifically bind to an antigen IL-7R. It has been shown that certain fragments of a full-length antibody can perform the antigen-binding function of the antibody. Examples of binding fragments encompassed within the term "antigen-binding fragment" of an antibody include (i) a Fab fragment, a monovalent fragment consisting of the VL, VH, CL and CH1 domains; (ii) a F(ab)2 fragment, a bivalent fragment comprising two Fab fragments linked by a disulfide bridge at the hinge region; (iii) an Fd fragment consisting of the VH and CH1 domains; (iv) a Fv fragment consisting of the VL and VH domains of a single arm of an antibody; (v) a dAb fragment which consists of a VH domain; and (vi) an isolated complementarity determining region (CDR) capable of specifically binding to an antigen, or any fusion proteins comprising such antigen-binding fragments. Furthermore, although the two domains of the Fv fragment, VL and VH, are coded for by separate genes, they can be joined, using recombinant methods, by a synthetic linker that enables them to be made as a single chain protein in which the VL and VH regions pair to form monovalent molecules (known as single chain Fv (scFv or scFvs). Also within the invention are antigen-binding molecules comprising 5 a VH and/or a VL. In the case of a VH, the molecule may also comprise one or more of a CH1, hinge, CH2, or CH3 region. Such single chain antibodies are also intended to be encompassed within the term "antigen-binding fragment" of an antibody. Other forms of single chain antibodies, such as diabodies, are also encompassed. Diabodies are bivalent, bi-specific antibodies in which VH and VL domains are expressed on a single polypeptide chain, but using a linker that is too short to allow for pairing between the two domains on the same chain, thereby forcing the domains to pair with complementary domains of another chain and creating two antigen-binding sites.

These antibody fragments are obtained using conventional techniques known to those of skill in the art, and the fragments are screened for utility in the same manner as are intact antibodies.

In some embodiments, the IL-7R modulator (i.e. IL-7R antagonist or IL-7R agonist) is a polypeptide. The term "polypeptide" means herein a polymer of amino acids having no specific length. Thus, peptides, oligopeptides and proteins are included in the definition of "polypeptide" and these terms are used interchangeably throughout the specification, as well as in the claims. The term "polypeptide" does not exclude post-translational modifications that include but are not limited to phosphorylation, acetylation, glycosylation and the like.

In some embodiments, the functional equivalent of IL-7R is fused to a heterologous polypeptide to form a fusion protein. As used herein, a "fusion protein" comprises all or part (typically biologically active) of a functional equivalent of the present disclosure operably linked to a heterologous polypeptide (i.e., a polypeptide other than the same polypeptide). Within the fusion protein, the term "operably linked" is intended to indicate that the functional equivalent of the present disclosure and the heterologous polypeptide are fused in-frame to each other. The heterologous polypeptide can be fused to the N-terminus or C-terminus of the functional equivalent of the present disclosure.

In one embodiment, the IL-7R modulator is an IL-7R antagonist, said IL-7R antagonist may be a polypeptide such as V7R-2E9 as disclosed in WO 2020/254827, a soluble IL-7R polypeptide as disclosed in WO 2010/017468, an anti-IL-7R antibody or antigen binding fragment thereof, such as N13B2-hVL6 antibody or antigen binding fragment thereof as disclosed in WO 2018/104483, C1GM or HAL403a antibody or antigen binding fragment thereof as disclosed in WO 2011/104687, 1A11.H3L4 antibody or antigen binding fragment thereof as disclosed in WO 2011/094259, other anti-IL-7R antibodies are disclosed in other publications such as WO 2013/056984 or WO 2015/189302.

In another embodiment, said IL-7R antagonist may be a small molecule inhibitor. In some embodiment, said IL-7R antagonist is an antisense oligonucleotide comprising no modified nucleoside or a modified nucleoside such as locked nucleic acids (LNA; e.g., oxyl-LNA, amino-LNA, thio-LNA), phosphorodiamidate morpholino oligomers (PMO), phosphorothioate (PS), 2'-0-mehyl (2'-Ome), 2'-fluro (2'-fluoro (2'-F)), or 2'-methoxyethyl (2'-MOE) derivatives. Antisense oligonucleotides are single-stranded polynucleotide molecules, usually 13-25 nucleotides in length that hybridize to complementary RNA, inhibit mRNA function, and prevent protein synthesis through accelerated mRNA degradation by RNase H or steric blockade.

In some embodiment, said IL-7R antagonist is a silencing RNA molecule (siRNA). siRNA refers to nucleotides of 19-23 bases in length which incorporate into an RNA-induced silencing complex in order to guide the complex to homologous endogenous mRNA for cleavage and degradation of the target mRNA.

In some embodiments, said IL-7R antagonist is a short hairpin RNA (shRNA). shRNA refers to an artificial double- stranded oligonucleotide with a tight hairpin turn that can be used to silence target gene expression via RNA interference.

In some embodiments, said IL-7R antagonist is a microRNA (miRNA) molecule microRNAs are small non-coding RNAs belonging to a class of regulatory molecules found in plants and animals that control gene expression by binding to complementary sites on target mRNA transcripts.

In some embodiments, said IL-7R antagonist is a ribozyme. A ribozyme is an enzymatic nucleic acid (e.g., RNA) molecule that can cleave and/or ligate to a part of itself or to other separate nucleic acid molecules, and therefore down regulate expression of targeted RNA.

In one embodiment, the IL-7R modulator is an IL-7R agonist, such as IL-7 (Interleurkin-7 cytokine) or TSLP (Thymic Stromal Lymphopoietin cytokine). IL-7 exhibits inhibitory effects in tumors such as glioma, melanoma, lymphoma, leukemia, prostate cancer, and glioblastoma; and administration of IL-7 in murine tumor models has shown to decrease cancer cell growth. IL-7 has also been shown to have potential in the treatment of lymphopenias, septic shock, and infectious disease as well enhancement of response to vaccination. IL-7 is currently being studied to prevent or reverse lymphopenia associated with COVID-19. As IL-7R signaling pathway has been implicated in autoimmune disease, inflammatory disease and cancer disease, therefore therapeutic targeting of said pathway is expected to have clinical benefit in said diseases. Administration of recombinant IL-7 has been found to be well tolerated in clinical trials.

In one embodiment, the IL-7R modulator (i.e. IL-7R antagonist or IL-7R agonist) is an aptamer. Aptamers are oligonucleotide or oligopeptide sequences with the capacity to recognize virtually any class of target molecules with high affinity and specificity. Aptamers are a class of molecule that represents an alternative to antibodies in term of molecular recognition.

The IL-7R modulator, i.e. IL-7R antagonist or IL-7R agonist, may be administered by any means known to those skilled in the art, including, parenteral administration. As used herein, "parenteral administration" of an IL-7R modulator includes any route of administration characterized by physical breaching of a tissue of a subject and administration of the pharmaceutical composition through the breach in the tissue, thus generally resulting in the direct administration into the blood stream, into muscle, or into an internal organ. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intravenous, intratumoral, and intrasynovial injection or infusions. Regional perfusion is also contemplated. Particular embodiments include the intravenous and the subcutaneous routes.

It will be appreciated that the specific dosage of IL-7R antagonist or agonist administered in any given case may vary with the type and severity of the condition to be alleviated, and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the embodied composition. Further, the dosage regimen with the compositions of this invention may be based on a variety of factors, including the type of disease, the age, weight, sex, medical condition of the patient, the severity of the condition, the route of administration, and the particular antibody employed. Thus, the dosage regimen can vary widely, but can be determined routinely using standard methods. For example, doses may be adjusted based on pharmacokinetic or pharmacodynamic parameters, which may include clinical effects such as toxic effects and/or laboratory values. Determining appropriate dosages and regimens are well-known in the relevant art and would be understood to be encompassed by the skilled artisan.

Suitable dosage ranges for IL-7R modulator, i.e. IL-7R antagonist or IL-7R agonist, may be of the order of several hundred micrograms of the agent with a range from about 0.001 to 100 mg/kg, preferably with the range from about 0.1 to 20 mg/kg, more preferably from about 1 to 12 mg/kg.

The invention will now be exemplified with the following examples, which are not limitative.

### EXAMPLES

### IL-7R 4-gene signature

To identify the IL-7R gene signature, the inventors analyzed differentially expressed genes (DEGs) (Fold Change>1.7, p-value = 0.05) from peripheral blood mononuclear cells (PBMCs) incubated with or without IL-7 (5ng/mL) at 3 hours and 18 hours and identified a set of dysregulated genes.

Freshly isolated PBMCs from healthy controls (HCs) (500.000 cells) were stimulated with 5 ng/mL of recombinant human IL-7 (R&D Systems) for 3h (H3) and 18h (H18) at 37°C. Cells were stored at -80°C in RLT buffer (Qiagen) containing 1% β-mercaptoethanol. Total RNA was purified using RNeasy mini kit Qiagen (cat 74104). RNA quantification and quality control were performed using Qubit (Thermofisher), and Tapestation (Agilent) assays. SMARTer Ultra Low kit (Takara Bio/Clontech) was used to synthesize the first strand of the cDNA based on the RT properties using polydT primers. The full-length cDNA was then amplified by Long-distance PCR (LD-PCR) before being quantified with the High Sensitivity Qubit kit and quality controlled with the High Sensitivity chip in a BioAnalyzer machine. Illumina-compatible sequencing libraries were constructed using Nextera XT kit (Illumina). The Nextera technology uses a modified transposase to fragment and ligate adapters in a unique step called "tagmentation". The libraries were then amplified by PCR using indexed primers allowing samples to be pooled. Quality control and quantification estimations for each library were done after the last purification using High Sensitivity chip (Tapestation). Sequencing was carried out as paired-end 2x150pb configuration (Illumina sequencer) with 50M paired-end reads guaranteed per sample.

Following a general sequence quality control using FastQC, fastq files were trimmed with Cutadapt in paired-end mode to clean up sequences containing sequencing adapters. Trimmed reads were mapped to the human hg38 reference genome using the STAR aligner. Aligned data were evaluated for quality using several quality metrics (e.g., mapping rate, multimapped reads, junction). Samtools idxstats (http://www.htslib.org/doc/samtools.html) was used to quantify the reads per chromosome and CollectRnaSeqMetrics from PicardTools (https://broadinstitute.github.io/picard/) was used to calculate the proportion of reads mapped on coding, intronic, intergenic, ribosomic and UTR regions. Read counts per gene were generated using featureCount based on the last GRCh38 annotations. Read counts were normalized by the variance stabilizing transformation vst function from DESeq2 R package. Up to 39,282 genes were detected in the data. Those with 0 count over all the samples or having an expression level below 1 in more than 95% were filtered out. At the end, the obtained RNA-Seq data comprised 15,064 genes.

To identify groups with genes differentially expressed, a linear model (ImFit function from limma R package) was performed on the vst transformation gene expression dataset. Genes with absolute fold change (FC) > 1.7 and Benjamini and Hochberg adjusted p-value < 0.05 were considered as differentially expressed genes (DEG). To capture early and late transcriptional changes mediated by IL-7, overlapping induced genes with consistent expression course from both DEG at 3 hours (H3) and 18 hours (H18) were retained.

Then the DEG observed on PBMCs experiments were applied on whole blood (WB) sample RNAseq of the PRECISESADS IMI cohort. The cross-sectional cohort of the PRECISESADS IMI project was recruited in 18 institutions from 9 European countries. Whole blood samples were collected in Tempus tubes and RNA was extracted using Tempus technology (Applied biosystems). The HiSeq2500 instrument and the TruSeq Stranded mRNA HT kit (Illumina) were used to performed 50 cycles of single-read sequencing. Preprocessing, quality controls of each run and cutting adapters were achieved using respectively bcl2fastq, FastQC tools 21 and Cutadapt22. Reads were aligned using STAR v2.5.2b23 to the UCSC Homo sapiens reference genome hg19. The quantification data was carried out using RSEM v1.2.3124 and after removing genes presenting less than 5 reads, 16.476 genes were kept for the analysis.

By analyzing the modulation of the IL-7R gene signature, the inventors identified 4 genes, namely *IKZF4, KIAA0040, PGAP1* and SOS1, that significantly increased in whole blood of patients suffering from autoimmune diseases (AIDs): primary Sjögren's syndrome (pSS, n=341), systemic lupus erythematosus (SLE, n=405), rheumatoid arthritis (RA, n=309), systemic sclerosis (SSc, n=328), mixed connective tissue disease (MCTD, n=91), undifferentiated connective tissue disease (UCTD, n=133) and primary anti-phospholipid syndrome (PAPS, n=92)), when compared to healthy control subjects (HCs, n=555) (Figure 1).

To evaluate the efficiency of this signature, the inventors established a z-score for each gene calculated as follows: z = (x-µ)/σ, where x is the value of the test sample, µ is the control population mean, and σ is the control population standard deviation. The final value is the mean of the 4-gene z-scores.

The inventors demonstrated a significant increase of the 4-gene z-score in AIDs patients' whole blood compared to the 4-gene z-score determined in healthy control subjects (Figure 1).

Table 1 shows a higher sensitivity in the z-score values and a significant difference indicated by the FDR values when considering the IL-7R 4-gene signature than for each gene separately. This applies for all autoimmune diseases together and for each disease of the cross-sectional cohort of the PRECISESADS IMI project, namely MCTD, PAPS, RA, pSS, SLE, SSc and UCTD.

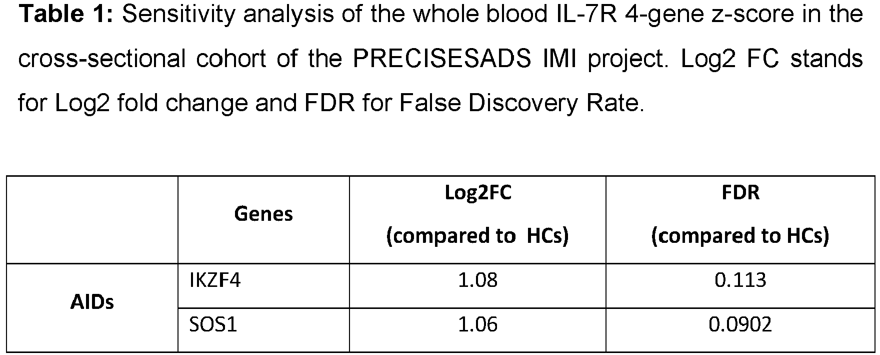

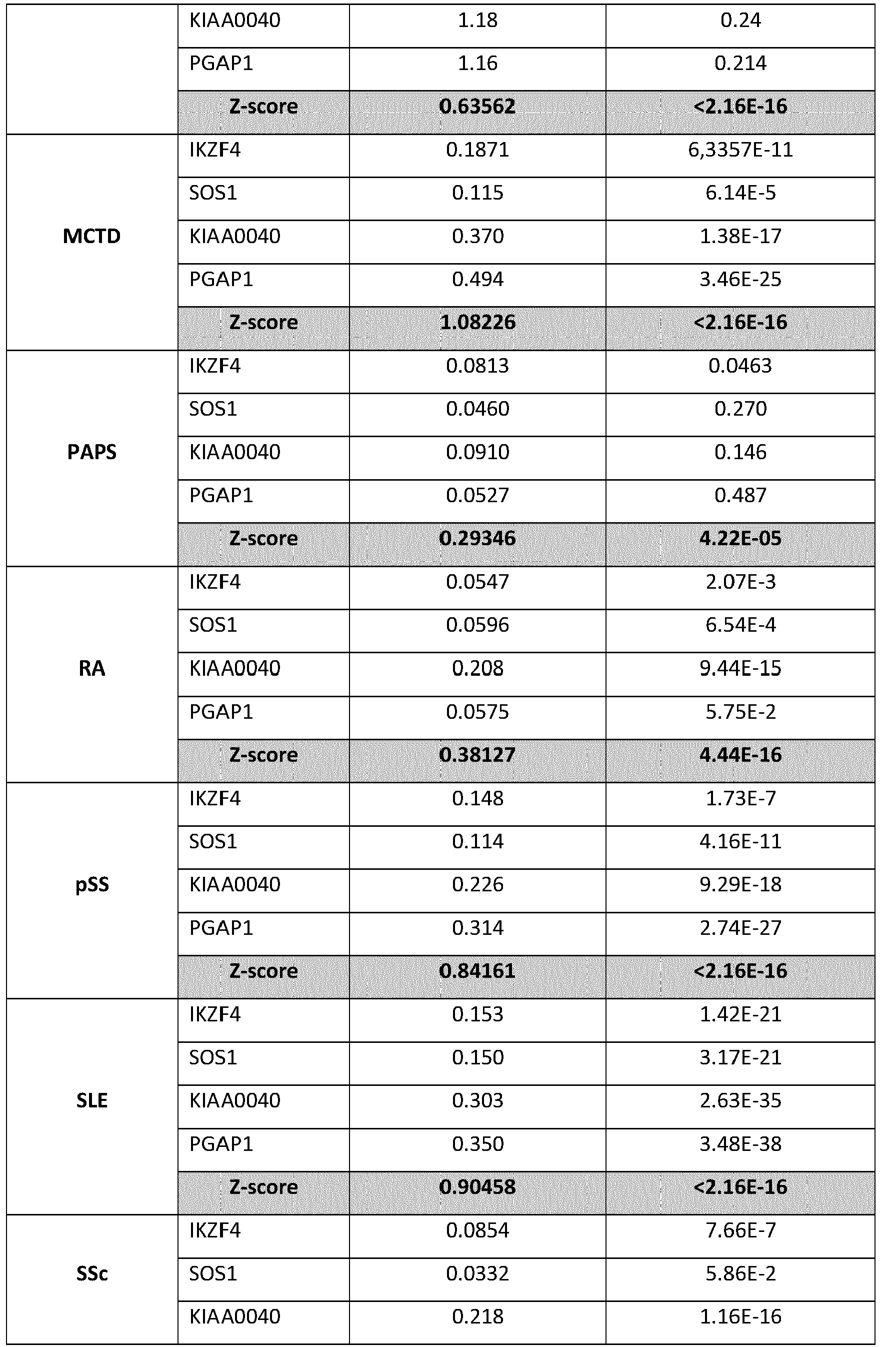

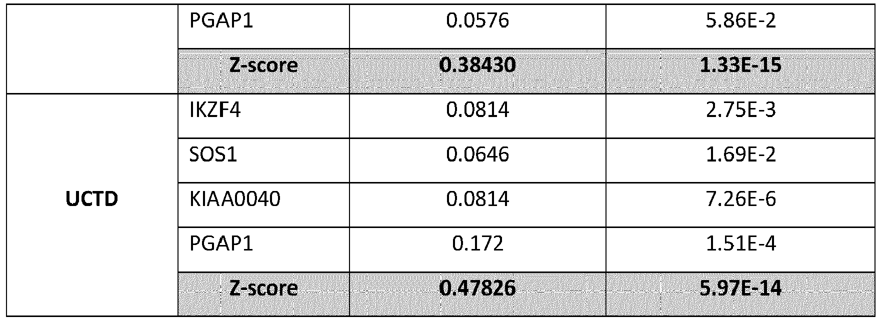

Altogether, these results demonstrate that this 4-gene IL-7R signature allows the identification of AIDs patients with high involvement of IL-7R signaling pathway compared to healthy control subjects (Figure 2).

### IL-7R 4-gene signature associates with biological measurements and/or disease activity

Generally, access to blood is easier and well accepted by patients. The inventors demonstrated that the z-score of the 4-gene signature in whole blood correlated with disease activity and/or biological activities in AIDs patients (Table 2a to Table 2e).

A two-step approach was adopted for the determination of different autoantibodies. Firstly, a screening of anti-ENA (extractable nuclear antigen) using magnetic particles coupled with a mixture of nuclear autoantigens was performed on the chemiluminescent immuno-analyser IDS-ISYS (Immunodiagnostic, Boldon, UK). In case of positive findings, a second panel was then triggered in order to detect the specific autoantibodies targeting said autoantigens: Smith antibody (SM), anti-ribonucleoprotein antibodies (U1-RNP), anti-topoisomerase antibodies (SCL70), anti-Sjögren Syndrome A (SSA) 52kDa or anti-Ro52 (SSA.52), anti-Sjögren Syndrome A 60kDa or anti-Ro60 (SSA.60) and anti-Sjögren Syndrome B antibodies (SSB)). Along with this strategy, anti-CCP2 and anti-dsDNA autoantibodies (respectively CCP2 and DNA) were also determined in all patients using the chemiluminescent immunoanalyzer IDS-ISYS (Immunodiagnostic, Boldon, UK). Furthermore, rheumatoid factor (RF) was determined regardless of the isotypes by turbidimetry with the SPA plus (The Binding Site).

The inventors applied this 4-gene z-score in whole blood samples from pSS patients (Table 2a). They demonstrated that the z-scores in patients positive for anti-SSA/Ro (both anti-SSA 60 kDa and anti-SSA 52 kDa), anti-SSB, anti-DNA, anti-SM and RF were significantly increased when compared to patients negative for each mentioned anti-autoantibody or to RF, as shown by the significant p-values.

Moreover, the disease activity of patients with pSS can be determined by the clinical scale ESSDAI (EULAR Sjögren's syndrome disease activity index). The inventors demonstrated that the z-score of the 4-gene signature in whole blood correlated with disease activity. Indeed, the expression of the 4-gene signature was significantly higher in patients with a high ESSDAI (above or equal to 5, corresponding to moderate to severe Sjögren patients), compared to patients having a low ESSDAI (value below 5) (Table 2a).

**Table 2a: Correlation of biological and clinical parameters with 4-gene z-score in pSS patients from PRECISESADS cohort**

| **pSS patients** | | n | *Mean* | *p-value* |
|---|---|---|---|---|
| ***AutoAbs*** | | | | |
| ***ENA*** | Positive | 222 | 0.922 | **1.1E-06** |
| | Negative | 33 | 0.219 | |
| ***SSA*** | Positive | 172 | 0.912 | **5.6E-06** |
| | Negative | 34 | 0.240 | |
| ***SSA.52*** | Positive | 144 | 0.984 | **3.5E-07** |
| | Negative | 43 | 0.287 | |
| ***SSA.60*** | Positive | 140 | 0.990 | **2E-07** |
| | Negative | 46 | 0.301 | |
| ***SSB*** | Positive | 80 | 0.949 | **0.042** |
| | Negative | 126 | 0.708 | |
| ***DNA*** | Positive | 9 | 1.387 | **0.026** |
| | Negative | 246 | 0.811 | |
| ***SM*** | Positive | 2 | 1.792 | **0.07** |
| | Negative | 203 | 0.797 | |
| ***RF*** | Positive | 110 | 0.990 | **0.013** |
| | Negative | 151 | 0.748 | |

| ***Clinical*** | | | | |
|---|---|---|---|---|
| ***ESSDAI*** | ESSDAI>=5 | 69 | 1.050 | **0.005** |
| | ESSDAI < 5 | 128 | 0.687 | |

The inventors also applied this 4-gene z-score in whole blood samples from SLE patients (Table 2b). In SLE patients, the expression of the 4-gene signature was also correlated with auto-antibodies' positivity, especially the most prevalent in this disease: anti-DNA, anti-ENA, anti-SSA (both anti-SSA 60 kDa and anti-SSA 52 kDa), anti-ribonucleoprotein antibodies (U1.RNP), anti-topoisomerase antibodies (SCL70), anti-Sm and autoantibodies against cyclic citrullinated peptide 2 (CCP2) as a significant p-value is observed when comparing positive and negative patients for each mentioned autoantibody.

**Table 2b: Correlation of biological parameters with 4-gene score in SLE patients from PRECISESADS cohort**

| **SLE patients** | | n | *Mean* | *p-value* |
|---|---|---|---|---|
| ***AutoAbs*** | | | | |
| ***CAM*** | Positive | 158 | 1.149 | **3.7E-07** |
| | Negative | 166 | 0.692 | |
| ***SSA*** | Positive | 91 | 1145 | **0.00034** |
| | Negative | 206 | 0.789 | |
| ***SSA.52*** | Positive | 64 | 1.143 | **0.0018** |
| | Negative | 224 | 0.805 | |
| ***SSA.60*** | Positive | 79 | 1.094 | **0.0032** |
| | Negative | 210 | 0.801 | |
| ***U1.RNP*** | Positive | 69 | 1.248 | **0.0007** |
| | Negative | 229 | 0.794 | |
| ***SCL70*** | Positive | 4 | 2.009 | **0.014** |
| | Negative | 294 | 0.884 | |
| ***DNA*** | Positive | 109 | 1.199 | **4.3E-05** |
| | Negative | 215 | 0.771 | |
| ***SM*** | Positive | 15 | 1.545 | **0.00097** |
| | Negative | 282 | 0.866 | |
| ***CCP2*** | Positive | 9 | 1.581 | **0.0087** |
| | Negative | 315 | 0.896 | |

In systemic sclerosis patients (SSc), the inventors demonstrated that the z-score of the 4-gene signature in whole blood is correlated with the positivity of the most prevalent antibodies in this disease, i.e. anti-ENA, as well as anti-SSA 52 kDa, anti-SSB and anti-topoisomerase antibodies (SCL70). Indeed, a significant p-value is observed when comparing positive and negative patients for each autoantibody (Table 2c).

**Table 2c: Correlation of biological parameters with 4-gene score in SSc patients from PRECISESADS cohort**

| **SSc patients** | | *n* | *Mean* | *p-value* |
|---|---|---|---|---|
| ***AutoAbs*** | | | | |
| ***CAM*** | Positive | 135 | 0.500 | **0.00039** |
| | Negative | 137 | 0.210 | |
| ***SSA*** | Positive | 40 | 0.664 | **0.0032** |
| | Negative | 223 | 0.288 | |
| ***SSA.52*** | Positive | 31 | 0.683 | **0.008** |
| | Negative | 226 | 0.288 | |
| ***SSB*** | Positive | 4 | 0.988 | **0.067** |
| | Negative | 265 | 0.342 | |
| ***SCL70*** | Positive | 94 | 0.478 | **0.013** |
| | Negative | 171 | 0.275 | |

Same observations were obtained in patients suffering from mixed connective tissue disease (MCTD), for which correlations between anti-ENA, anti-DNA and anti-RNP positivity and a high 4-gene z-score were observed, as demonstrated by the significant p-values, when comparing positive and negative patients for each autoantibody (Table 2d).

**Table 2d: Correlation of biological parameters with 4-gene score in MCTD patients from PRECISESADS cohort**

| **MCTD patients** | | *n* | *Mean* | *p-value* |
|---|---|---|---|---|
| ***AutoAbs*** | | | | |
| ***CAM*** | Positive | 55 | 1.223 | **0.0053** |
| | Negative | 16 | 0.490 | |
| | Negative | 63 | 0.975 | |
| ***DNA*** | Positive | 5 | 1.886 | **0.02** |
| | Negative | 66 | 0.995 | |
| ***U1.RNP*** | Positive | 48 | 1.179 | **0.02** |
| | Negative | 19 | 0.507 | |

In patients suffering from undifferentiated connective tissue disease (UCTD), the inventors demonstrated that a high 4-gene z-score in whole blood is also correlated with the presence of various autoantibodies such as anti-ENA, anti-SSA (both Ro52 and Ro60) and anti-SSB, as demonstrated by the significant p-values when comparing positive and negative patients for each autoantibody (Table 2e).

**Table 2e: Correlation of biological parameters with 4-gene score in UCTD patients from PRECISESADS cohort**

| **UCTD patients** | | *n* | *Mean* | *p-value* |
|---|---|---|---|---|
| ***AutoAbs*** | | | | |
| ***CAM*** | Positive | 31 | 0.926 | **0.00021** |
| | Negative | 64 | 0.370 | |
| ***SSA*** | Positive | 16 | 0.949 | **0.008** |
| | Negative | 75 | 0.362 | |
| ***SSA.52*** | Positive | 9 | 0.854 | **0.069** |
| | Negative | 80 | 0.475 | |
| ***SSA.60*** | Positive | 12 | 0.963 | **0.012** |
| | Negative | 77 | 0.443 | |
| ***SSB*** | Positive | 4 | 1.134 | **0.023** |
| | Negative | 88 | 0.506 | |

Collectively, these results demonstrate that the 4-gene IL-7R signature *(IKZF4, KIAA0040, PGAP1 and SOS1)* allows to discriminate AIDs patients from healthy control subjects. It also allows to discriminate patients in different groups either regarding their biological activities as measured by the presence of auto-antibodies and/or relevant proteins and/or regarding the severity of the disease as measured by appropriate clinical scales.

### IL-7R 5-gene signature

Biopsies are accepted for diagnostic purposes as in Sjögren's syndrome, ulcerative colitis or cancer, or for research purpose when the collection is not too much invasive as for cutaneous biopsies. Moreover, biopsies are routinely used to monitor early treatment response.

The inventors analyzed pSS patients' tissue biopsies from (i) two public transcriptomic datasets from parotid glands (GSE40611, GSE173808) and (ii) two public transcriptomic datasets from labial minor salivary glands (MSG) (GSE157159, GSE173808) (Table 3).

**Table 3: Details of transcriptomic datasets, wherein "pSS" stands for primary Sjögren's syndrome, "HC" stands for healthy control subjects, "SC" for sicca control patients and "MSG" stands for minor salivary glands:**

| **NCBI code** | **Tissue** | **Population** | **Contrast** | **Publication** |
|---|---|---|---|---|
| **GSE17380 8** | Parotid gland | 21 pSS | pSS vs SC | https://www.ncbi.nlm.nih.gov/ge o/query/acc.cgi?acc=GSE173808 |
| | | 16 SCs | | |
| **GSE17380 8** | MSG (labial) | 33 pSS | pSS vs SC | https://www.ncbi.nlm.nih.gov/ge o/query/acc.cgi?acc=GSE173808 |
| | | 17 SCs | | |
| **GSE15715 9** | MSG | 10 pSS | pSS vs HC | https://www.ncbi.nlm.nih.gov/ge o/query/acc.cgi?acc=GSE157159 |
| | | 10 HCs | | |
| **GSE40611** | Parotid gland | 7 pSS | - pSS vs SC | https://www.ncbi.nlm.nih.gov/ge o/query/acc.cgi?acc=GSE40611 |
| | | 14 SCs | - pSS vs HC | |
| | | 9 HCs | - SC vs HC | |

Using the meta-datasets, a linear model was performed to identify score differentially expressed groups, resulting p-values were adjusted for multiple hypothesis testing and filtered to retain DEGs with a False Discovery Rate (FDR) adjusted p-value < 0.05 and a |Fold-Change (FC)| ≥ 1.3.

The inventors determined an IL-7R 5-gene signature namely *IL-7R, PCED1B, TNFSF8, ADAM19* and *MYBL1.* These 5 genes were markedly and consistently increased in the parotid and minor salivary glands biopsies of pSS patients, compared to Sicca patients (SCs) or healthy controls (HCs). To evaluate the efficiency of this 5-gene signature *(IL-7R, PCED1B, TNFSF8, ADAM19 and MYBL1),* the inventors established a 5-gene signature z-score.

The z-score for each gene has been calculated as follows: z = (x-µ)/σ, where x is the value of the test sample, µ is the control population mean, and σ is the control population standard deviation. The final value is the mean of the 5-gene z-scores.

The inventors demonstrated a significant increase of the 5-gene signature z-score in pSS patients tissues compared to either healthy control subjects' or Sicca patients' tissues (Figure 3), indicating this 5-gene signature allows the discrimination of pSS patients from healthy control subjects or Sicca patients. Moreover, with the IL-7R 5-gene signature z-score, a higher sensitivity was observed in comparison to the analysis of each gene separately, with a significant difference, as shown in the Table 4 below.

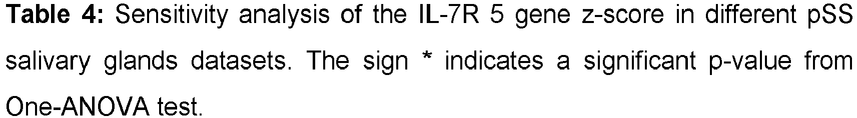

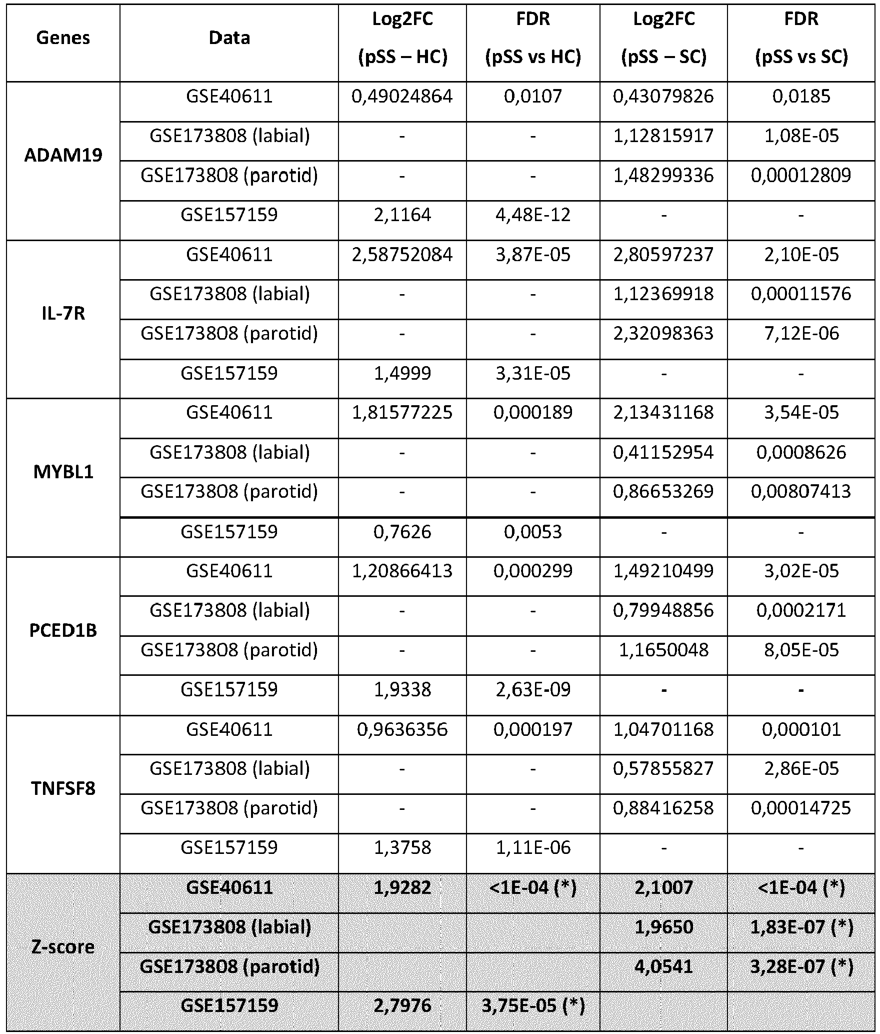

### IL-7R 5-gene signature associates with disease activity

Since increased lymphocytic infiltrates, particularly B cell infiltrates, have been associated with higher focus scores in pSS, predictive molecular signatures of several cell types based on methods from Galon *et al.* and Becht *et al.* to evaluate the abundance of tissue-infiltrating immune cell populations were applied. An « immune infiltrate score » reflecting each signature's presence was computed from the expression of the genes in the signature. Scores were computed as the mean expression of the genes constituting the signature. Based on the distribution of the enrichment density of B and T cell scores, individuals from each tissue dataset were segregated into a high and low infiltration status.

The inventors applied the 5-gene signature z-score *(IL-7R, PCED18, TNFSF8, ADAM19 and MYBL1)* on pSS patients' tissue datasets, according to their status of high or low infiltration of B cells (Figure 4A) or their status of high or low infiltration of total lymphocytes (Figure 4B).

All pSS tissue datasets from patients, either with high or low infiltrated B cells or with high or low infiltrated total lymphocytes, showed significant increase of the 5-gene signature z-score, compared to healthy control subjects (HC) or sicca control patients (SC). Moreover, this 5-gene signature z-score was particularly increased in patients with high B cells and/or high total lymphocytes enrichments in all data sets (Figure 4A and Figure 4B). The 5-gene signature of the present invention would thus further allow to discriminate pSS patients according to the degree of infiltrates of B cells or total lymphocytes, a high expression of the 5-gene signature z-score in tissue correlating with a high degree of B cells and total lymphocytic infiltrates.

The inventors analyzed also synovial tissue of rheumatoid arthritis (RA) patients from five public transcriptomic datasets (Table 5).

**Table 5: Details of transcriptomic datasets, wherein "RA" stands for Rheumatoid arthritis, "OA" for OsteoArthritis and "HC" stands for healthy control subjects:**

| **NCBI code** | **Tissue** | **Population** | **Contrast** |
|---|---|---|---|
| **GSE77298** | Synovial tissue | 23 samples: | HC vs RA |
| | | 16 samples of RA patients and 7 samples of HC. | |
| **GSE1919** | Synovial tissue | 15 samples: | HC vs RA |
| | | 5 HC, 5 RA patients and 5 OA patients. | |
| **GSE55457** | Synovial tissue | 33 samples: | HC vs RA |
| | | 10 HC, 13 RA patients and 10 OA patients. | |
| **GSE55235** | Synovial tissue | 30 samples: | HC vs RA |
| | | 10 HC, 10 RA patients and 10 OA patients. | |
| **GSE89408** | Synovial tissue | 216 samples: | HC vs RA |
| | | Arthralgia patients (n= 10), Arthritis patients (n=6), OA patients (n=21), RA patients (n=152) and healthy control subjects (n=27). | |

The inventors demonstrated a significant increase of the 5-gene signature z-score in synovial tissue of rheumatoid arthritis patients compared to healthy control subjects (Figure 5), indicating this 5-gene signature allows the identification of RA patients from control subjects, and thus showing the specific dysregulation of the IL-7R signaling pathway in such disease.

Skin biopsies from 6 different data sets of patients suffering from different subtypes of cutaneous lupus erythematosus (CLE) were also analyzed (Table 6).

**Table 6: Details of transcriptomic datasets, wherein "CDLE" stands for chronic discoid lupus erythematosus, "CCLE" stands for Chronic Cutaneous Lupus Erythematosus, "sCLE" for subacute cutaneous lupus erythematosus and "HC" for healthy control subjects:**

| **NCBI code** | **Tissue** | **Population** | **Contrast** |
|---|---|---|---|
| **GSE52471** | skin | 7 active CDLE patients and 13 HC | CDLE vs HC |
| **GSE81071** | skin | 26 CDLE patients, 23 sCLE patients and 7 HC | CDLE vs sCLE vs HC |
| **GSE95474** | skin | 6 CDLE patients, 5 sCLE patients and 5 HC | CDLE vs sCLE vs HC |
| **GSE109248** | skin | 6 CCLE patients, 12 sCLE patients and 14 HC | CCLE vs sCLE vs HC |
| **GSE72535** | skin | 9 CDLE patients and 8 HC | CDLE vs HC |

Same observations were obtained in skin biopsies of both CDLE and sCLE patients since a significant increase of the 5-gene signature z-score in the skin of these patients were observed in comparison to healthy control subjects' tissue (Figure 6).

The inventors applied the 5-gene signature z-score (*IL-7R, PCED18, TNFSF8, ADAM19 and* MYBL1) on RA and CLE patients' tissue datasets, according to their status of high or low infiltration of lymphocytes in synovial membrane tissue of RA patients (Figure 7A) or in CLE skin tissue (Figure 7B). As for pSS patients, the 5-gene signature z-score was particularly increased in patients with high lymphocyte infiltrates in all studied data sets.

All these data demonstrated the relevance of the 5-gene signature z-score in different tissues of patients suffering from different AIDs as it allows to identify patients with a high IL-7R pathway involvement compared to control subjects.

Moreover, the 5-gene signature z-score enables the stratification or discrimination of patients suffering from IL-7R-mediated disease according to disease activity, as a higher 5-gene signature z-score is associated with a higher disease activity, as shown here by the observation of a higher lymphocytes' infiltration.

Finally, the 5-gene signature z-score can also be used for monitoring a disease activity over time or for monitoring the treatment efficacy with an IL-7R modulator, by following the 5-gene z-scores of CLE patients according to their level of lymphocytes' infiltration.

### Application of IL-7R gene signature for assessing the agonistic effect or antagonistic effect on IL-7R activity and for monitoring IL-7R activity modulation

By applying the same methodology used for IL-7R signatures identification, the inventors evaluated the modulation of the IL-7R signature in the presence of an anti-IL-7R antagonist. PBMCs were stimulated with IL-7 (5ng/mL) during 3 hours (H3) and 18 hours (H18). Prior to the IL-7 stimulation and the induction of the IL-7R pathway, PBMCs were incubated for 30 min at 37°C with or without an IL-7R antagonist (N13B2-hVL6) at 10 µg/mL.

The inventors demonstrated a clear and significant overexpression of IL-7R 4-gene signature in PBMCs stimulated by IL-7 compared to unstimulated PBMCs (Figure 8), both at 3 and 18 hours after incubation. This indicates this 4-gene signature is relevant for assessing agonistic activity of IL-7R pathway.

Moreover, in presence of the IL-7R antagonist, an inhibition of the 4-gene signature was significantly observed at 18 hours, and not at 3 hours, indicating a time-dependent inhibition by the IL-7R antagonist. Consequently, this 4-gene signature is relevant for demonstrating the antagonistic activity of an IL-7R modulator and for assessing modulation of IL-7R pathway activity in a time-dependent fashion (Figure 8).

In order to give robustness to the previous observation, the 4-gene signature was evaluated by RT-qPCR, using two other IL7R antagonist antibodies, namely clones 1A11.H3L4 (indicated here as Ab1) and clone HAL403a (indicated here as Ab2). RT-qPCR is a useful method already in place in most laboratories as it allows to quantify the expression of few genes rapidly and in a cost-effective manner, thus is well suited for a routinely detection or monitoring a drug or a clinical effect, for instance.

Freshly isolated PBMCs from healthy controls (HCs) (500.000 cells) were stimulated with 5 ng/mL of recombinant human IL-7 (R&D Systems) for 24h at 37°C. Prior to the IL-7 stimulation and the induction of the IL-7R pathway, PBMCs were incubated for 30 min at 37°C with or without each IL-7R antagonist, Ab1 or Ab2 at 10 µg/mL.

All samples were lysed and homogenized using QIAshredder (Qiagen # 79654) in the adapted RLT Lysis Reagent buffer (Qiagen). Total RNA was extracted using the RNeasy Mini Kit (Qiagen # 74106), with the optional on-column DNAse I digestion. Upon extraction, total RNA samples were quantified on the Nanodrop^{™} 2000 spectrophotometer (ThermoFisher Scientific^{™}) and analyzed on the TapeStation 4150 using RNA ScreenTape (Agilent Technologies) to determine RNA quality (RIN).

A real time quantification analysis (RT-qPCR) of *IKZF4, KIAA0040, PGAP1* and *SOS1* genes was performed and associated with housekeeping genes (genes of reference). Total RNA was reversed transcribed into cDNA using random primers, dNTPS, RNAse inhibitor and multiScribe RT enzyme from the High Capacity cDNA Reverse Transcription kit (Applied biosystems^{™} #4368813).

Specific target amplification of cDNA was carried out with the pool of the TaqMan^{™} Gene Expression Assays (0.2X, Life Technologies) and TaqMan Preamp Master Mix 2X (Life Technologies). Real-time PCR was conducted on Fluidigm 96.96 Dynamic Arrays using the BioMark^{™} HD system. All samples were analysed in duplicate for quantification of the 4 targets genes mRNA and 12 housekeeping genes. Ct values were calculated using the BioMark Real-Time PCR Analysis software (Fluidigm) with the linear derivative baseline subtraction method and a user calculated threshold (set at 0.01). Quantifiable expression is defined by Ct values of 24 or lower, and undetectable expressions by Ct value over 40.

The selection of the most stable reference genes for normalization (CTCF and EP300) was evaluated using geNorm algorithm.

The technical replicates (n = 2) of the CT values were combined by geometric mean, and then were firstly normalized to endogenous controls by subtracting, for each sample, the CTCF and EP300 gene expression level mean (calcul of -dCt) and secondly normalized to an internal well plate calibrator (Calcul of -ddCt). Finally, the difference in the ddCt (calcul of - dddCt) was calculated between each sample and the mean of the control group (medium condition). The relative gene expression of each target and each sample was calculated as 2^{-dddCt}.

To identify genes differentially expressed, a mixed linear model (lme function from nlme R package) was performed on the -ddct gene expression dataset. Genes with absolute fold change (FC) > 1.3 and Benjamini and Hochberg adjusted p-value < 0.05 were considered as differentially expressed genes (DEG).

The results are shown in Figure 9. The inventors demonstrated a clear and significant overexpression of IL-7R 4-gene signature in PBMCs stimulated by IL-7, compared to unstimulated PBMCs, confirming the relevance of the 4-gene signature for assessing agonistic activity of IL-7R pathway and by using RT-qPCR method.

Moreover, in presence of the two IL-7R antagonists, an inhibition of the 4-gene signature was significantly observed, confirming the relevance of this signature for assessing the antagonistic activity of an IL-7R modulator and by using RT-qPCR method (Figure 9).

Altogether these results demonstrate that the 4-gene signature (*IKZF4, KIAA0040, PGAP1 and SOS1)* accurately measures the increase or decrease of the IL-7R pathway activity, allowing the monitoring of any modulation of the IL-7R pathway.

Collectively, both signatures (4-gene and 5-gene signatures) are useful for discrimination of patients suffering from IL-7R-mediated disease, for identifying a patient who would be responsive to a treatment with an IL-7R modulator, or for monitoring a treatment with an IL-7R modulator.

## Claims

1. A method of identification of a patient likely suffering from a disease with a pathological increase or decrease of the IL-7R signaling pathway comprising the steps of:
a) determining the gene expression profile in a biological sample of said patient of at least 2, 3 or 4 genes selected from the group consisting of: IKZF4, KIAA0040, PGAP1 and SOS1 genes, or
b) determining the gene expression profile in a biological sample of said patient of at least 2, 3, 4 or 5 genes selected from the group consisting of: IL-7R, PCED1B, TNFSF8, ADAM19 and MYBL1 genes, and
c) comparing the gene expression profile determined in a) or b) to a reference gene expression profile of said genes,
wherein respectively a higher or lower gene expression profile of said genes in said patient sample as compared to the reference gene expression profile of said genes is indicative that the patient is likely suffering from a disease respectively associated with an increase or decrease of the IL-7R signaling pathway.

2. The method according to claim 1, wherein said biological sample is a liquid sample, a tissue sample or a cell sample.

3. The method according to claim 2, wherein said liquid sample is a whole blood sample or synovial fluid, wherein said tissue sample is salivary gland biopsy, parotid gland biopsy, skin biopsy, synovial tissue biopsy or kidney biopsy, and wherein said cell sample is peripheral blood mononuclear cells.

4. The method according to claim 1 to 3 wherein said disease associated with an increase or decrease of the IL-7R signaling pathway is selected from the group consisting of an autoimmune disease, an inflammatory disease, a cancer disease and an infectious disease.

5. The method according to claim 4 wherein the autoimmune disease or inflammatory disease is Sjogren's syndrome or Sjogren's disease, systemic lupus erythematosus, rheumatoid arthritis, sclerosis, psoriasis, intestinal bowel disease or ulcerative colitis.

6. The method according to claim 4 wherein the cancer disease is T-cell acute lymphoblastic leukemia, B-cell acute lymphoblastic leukemia, chronic lymphocytic leukemia, T-cell prolymphocytic leukemia and Hodgkin's lymphoma.

7. A method of identification of a patient likely responsive to treatment with an IL-7R modulator, i.e IL-7R agonist or IL-7R antagonist, prior to said treatment by:
a) determining in a biological sample of said patient, whether the patient's gene expression profile of at least 2, 3 or 4 genes selected from the group consisting of: IKZF4, KIAA0040, PGAP1 and SOS1 genes is higher or lower compared to a reference gene expression profile, or
b) determining in a biological sample of said patient, whether the patient's gene expression profile of at least 2, 3, 4 or 5 genes selected from the group consisting of: IL-7R, PCED1B, TNFSF8, ADAM19 and MYBL1 genes is higher or lower compared to a reference gene expression profile,
wherein a higher or lower gene expression profile determined in a) or b) is indicative that the patient is likely responsive to treatment respectively with an IL-7R antagonist or with an IL-7R agonist.

8. The method according to claim 7, wherein said biological sample is a liquid sample, a tissue sample or a cell sample.

9. The method according to claim 8, wherein said liquid sample is a whole blood sample or synovial fluid, wherein said tissue sample is salivary gland biopsy, parotid gland biopsy, skin biopsy, synovial tissue biopsy or kidney biopsy, and wherein said cell sample is peripheral blood mononuclear cells.

10. A method for evaluating the therapeutic response to an IL-7R modulator of a patient treated with said IL-7R modulator, i.e. respectively with an IL-7R agonist or IL-7R antagonist, comprising the steps of:
a) determining in a biological sample of said patient the gene expression profile of at least 2, 3 or 4 genes selected from the group consisting of: IKZF4, KIAA0040, PGAP1 and SOS1 genes, or
b) determining in a biological sample of said patient the gene expression profile of at least 2, 3, 4 or 5 genes selected from the group consisting of: IL-7R, PCED1B, TNFSF8, ADAM19 and MYBL1 genes, and
c) comparing said gene expression profile to a gene expression profile determined in the same patient before said treatment or earlier during said treatment,
wherein respectively a higher or lower gene expression profile is indicative that the patient is likely responsive to the treatment with respectively the IL-7R agonist or the IL-7R antagonist.

11. The method according to claim 10, wherein said biological sample is a liquid sample, a tissue sample or a cell sample.

12. The method according to claim 11, wherein said liquid sample is a whole blood sample or synovial fluid, wherein said tissue sample is salivary gland biopsy, parotid gland biopsy, skin biopsy, synovial tissue biopsy or kidney biopsy, and wherein said cell sample is peripheral blood mononuclear cells.

13. A method for evaluating the therapeutic response to an IL-7R modulator of a patient treated with said IL-7R modulator, respectively with an IL-7R agonist or IL-7R antagonist, comprising the steps of:
a) determining in a biological sample of said patient the gene expression profile of at least 2, 3 or 4 genes selected from the group consisting of: IKZF4, KIAA0040, PGAP1 and SOS1 genes, or
b) determining in a biological sample of said patient the gene expression profile of at least 2, 3, 4 or 5 genes selected from the group consisting of: IL-7R, PCED1B, TNFSF8, ADAM19 and MYBL1 genes, and
c) comparing said gene expression profile to a gene expression profile determined in the patient before treatment or earlier during the treatment,
wherein respectively a lower or higher gene expression profile is indicative that the patient is likely not responsive to the treatment with respectively the IL-7R agonist or the IL-7R antagonist.

14. A method for evaluating the therapeutic response to an IL-7R modulator of a patient treated with said IL-7R modulator, i.e. with an IL-7R agonist or IL-7R antagonist, comprising the steps of:
a) determining in a biological sample of said patient the gene expression profile of at least 2, 3 or 4 genes selected from the group consisting of: IKZF4, KIAA0040, PGAP1 and SOS1 genes, or
b) determining in a biological sample of said patient the gene expression profile of at least 2, 3, 4 or 5 genes selected from the group consisting of: IL-7R, PCED1B, TNFSF8, ADAM19 and MYBL1 genes, and
c) comparing said gene expression profile to a gene expression profile determined in the patient before treatment or earlier during the treatment,
wherein an unchanged gene expression profile is indicative that the patient is likely not responsive to the treatment with said IL-7R modulator.

15. The method according to claim 13 or 14, wherein said biological sample is a liquid sample, a tissue sample or a cell sample.

16. The method according to claim 15, wherein said liquid sample is a whole blood sample or synovial fluid, wherein said tissue sample is salivary gland biopsy, parotid gland biopsy, skin biopsy, synovial tissue biopsy or kidney biopsy, and wherein said cell sample is peripheral blood mononuclear cells.

17. A method for predicting an IL-7R-mediated disease activity in a patient suffering from said disease, comprising the steps of:
a) determining, in a biological sample of said patient, the patient's gene expression profile of at least 2, 3 or 4 genes selected from the group consisting of: IKZF4, KIAA0040, PGAP1 and SOS1 genes, or
b) determining, in a biological sample of said patient, the patient's gene expression profile of at least 2, 3, 4 or 5 genes selected from the group consisting of: IL-7R, PCED1B, TNFSF8, ADAM19 and MYBL1 genes, and
c) comparing the gene expression profile determined in a) or b) to a gene expression profile of a representative group of patients suffering from said IL-7R-mediated disease,
wherein the patient's gene expression profile is indicative of a high disease activity when said profile is significantly higher than the gene expression profile of a representative group of patients with said IL-7R-mediated disease,
wherein the patient's gene expression profile is indicative of a low disease activity when said profile is significantly lower than the gene expression profile of a representative group of patients with said IL-7R-mediated disease.

18. The method according to claim 17, wherein said biological sample is a liquid sample, a tissue sample or a cell sample.

19. The method according to claim 18, wherein said liquid sample is a whole blood sample or synovial fluid, wherein said tissue sample is salivary gland biopsy, parotid gland biopsy, skin biopsy, synovial tissue biopsy or kidney biopsy, and wherein said cell sample is peripheral blood mononuclear cells.

20. The method according to claim 17, wherein said disease activity is correlated with a marker of an IL-7R-mediated disease.

21. The method according to claim 20, wherein said marker of an IL-7R-mediated disease is rheumatoid factor (RF), anti-cyclic citrullinated antibody (anti-CCP), antinuclear autoantibodies (ANA), anti-double-stranded DNA antibodies, antibodies to extractable nuclear antigens (ENA), antineutrophil cytoplasmic autoantibodies (ANCA) and/or said marker of an IL-7R-mediated disease is autoantibodies to Sjögren Syndrom Antigen A (SSA or Ro) 60 kDa (Ro/SSA 60), SSA 52 kDa (or Ro/SSA52), Sjögren Syndrom Antigen B (SSB or La), Jo-I, Smith antigen (Sm or SM), ribonucleoprotein (U1.RNP), topoisomerase (Scl-70) and centromer B peptide (CENB-P), and/or said marker of an IL-7R-mediated disease is lymphocytic infiltrates in tissues or a specific criteria comprised in a clinical scale such as EULAR Sjögren's syndrome disease activity index (ESSDAI) scale.

22. A method for selecting a compound likely effective in the treatment of a disease associated with respectively an increase or decrease of the IL-7R signaling pathway comprising the steps of:
a) culturing cells from a biological sample,
b) determining in said cell culture, the gene expression profile of at least 2, 3 or 4 genes selected from the group consisting of: IKZF4, KIAA0040, PGAP1 and SOS1 genes, and/or determining in said cell culture the gene expression profile of at least 2, 3, 4 or 5 genes selected from the group consisting of: IL-7R, PCED1B, TNFSF8, ADAM19 and MYBL1 genes,
c) comparing the determined gene expression profile to a reference gene expression profile, and
d) selecting a compound that induces respectively a lower or higher gene expression profile of said genes in said cell culture as compared to a reference gene expression profile.

23. The method according to claim 22, wherein such compound is an IL-7R modulator, either an IL-7R antagonist when the compound induces a lower gene expression profile of said genes in said cell culture as compared to a reference gene expression profile or an IL-7R agonist when the compound induces a higher gene expression profile of said genes in said cell culture as compared to a reference gene expression profile.

24. A kit comprising a set of reagents that specifically detects the gene expression profile of at least 2, 3 or 4 genes selected from the group consisting of: IKZF4, KIAA0040, PGAP1 and SOS1 genes, and/or at least 2, 3, 4 or 5 genes selected from the group consisting of: IL-7R, PCED1B, TNFSF8, ADAM19, MYBL1 genes.

25. The kit according to claim 24 wherein said reagents are primer pairs and/or probes specific of each gene.
